# FASCICULE DE BREVET EUROPEEN

(11) **EP 0 877 738 B1**
(45) Date de publication et mention de la délivrance du brevet: **14.05.2003**
(21) Numéro de dépôt: 96938306.6
(22) Date de dépôt: 14.11.1996
(51) Int. Cl.: C07D 233/72, C07D 491/10

(54) **PROCEDE DE PREPARATION DE DERIVES PHENYLIMIDAZOLIDINE**
VERFAHREN ZUR HERSTELLUNG VON PHENYLIMIDAZOLIN-DERIVATEN
PROCESS FOR THE PREPARATION OF PHENYLIMIDAZOLIDINE DERIVATIVES

(30) Priorité: 16.11.1995 FR 9513589
(43) Date de publication de la demande: 18.11.1998
(73) Titulaire: Aventis Pharma S.A., 92160 Antony (FR)
(72) Inventeur: BOUCHET, Rapha[l, F-93500 Pantin (FR); DELTHIL, Michel, F-93130 Noisy-le-Sec (FR); GUILMARD, Daniel, F-77680 Roissy-en-Brie (FR); MACKIEWICZ, Philippe, F-93190 Livry-Gargan (FR)
(86) Numéro de dépôt international: FR9601794
(87) Numéro de publication internationale: WO97018197

(56) Documents cités:
- EP-A- 0 436 426
- CHEMICAL ABSTRACTS, vol. 116, no. 19, 11 Mai 1992 Columbus, Ohio, US; abstract no. 187418w, P. CAMUS ET AL.: "Pharmacokinetics and Metabolisme of nilutamide in the isolated rat lung." page 7; colonne 1; XP002010118 & J. PHARMACOL. EXP. THER., vol. 259, no. 3, 1991, pages 1247-1255,
- CHEMICAL ABSTRACTS, vol. 109, no. 15, 10 Octobre 1988 Columbus, Ohio, US; abstract no. 128548w, S. HITOSHI ET AL.: "Preparation of bromoanilines as drug and agrochemical intermediates." page 644; colonne 1; XP002010119 & JP 62 298 562 A (NIPPON KAYAKU CO., LTD.) 25 Décembre 1987
- CHEMICAL ABSTRACTS, vol. 45, no. 7, 10 Avril 1951 Columbus, Ohio, US; J.F. SALELLAS ET AL.: "Nuclear halogenations with mono- and dibromodimethylhydantoin. I. Benzene derivatives." colonne 2873h; XP002010120 & ANALES ASOC. QUIM. ARGENTINA, vol. 38, 1950, pages 181-187,

## Description

La présente invention concerne un nouveau procédé de préparation de dérivés phényl imidazolidine.

Le document EP-A-0 436 426 enseigne un procédé de préparation de dérivés de la 1-phényl-imidazoline-2,5-dione.

La présente invention a ainsi pour objet un nouveau procédé de préparation des produits de formule (I) : 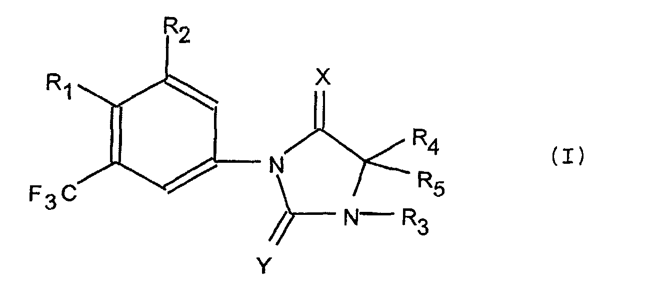 R₁ et R₂, identiques ou différents, sont choisis parmi l'atome d'hydrogène, les atomes d'halogène et les radicaux alkyle, alkényle, alkynyle, cyano, trifluorométhyle, amino, monoalkylamino et dialkylamino,
R₃ représente un atome d'hydrogène, un radical alkyle, éventuellement interrompu par un ou plusieurs atomes d'oxygène ou de soufre, un-radical phényle ou pyridyle, ces radicaux étant éventuellement substitués par un ou plusieurs radicaux choisis parmi les atomes d'halogène, le radical phényle, hydroxyle éventuellement estérifié, éthérifié ou protégé, alkoxy, cyano, trifluorométhyle, hydroxyalkyle, carboxy libre, estérifié, amidifié ou salifié, amino, mono ou dialkylamino, l'atome d'azote du radical pyridyle étant éventuellement oxydé,
R₄ et R₅
soit identiques ou différents, représentent un radical alkyle, éventuellement substitué par un ou plusieurs radicaux choisis parmi les radicaux hydroxyle éventuellement estérifié, éthérifié ou protégé, les atomes d'halogène et les radicaux alkylthio et phénylthio eux-mêmes éventuellement substitués par un ou plusieurs radicaux choisis parmi les atomes d'halogène et le radical hydroxyle,
soit forment ensemble le radical : 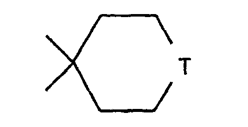 dans lequel T représente un atome d'oxygène ou de soufre,
X et Y, identiques ou différents, représentent un atome d'oxygène ou de soufre,
lesdits produits de formule (I) étant sous toutes les formes isomères racémiques, énantiomères ou diastéréoisomères possibles, ainsi que les sels d'addition avec les acides minéraux et organiques ou avec les bases minérales et organiques desdits produits de formule (I), caractérisé en ce que :
a) on prépare un produit de formule (A) : 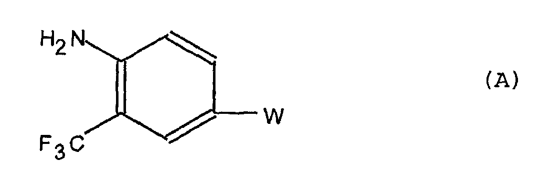 dans laquelle W représente un atome d'halogène ou un dérivé de l'hydantoïne de formule : 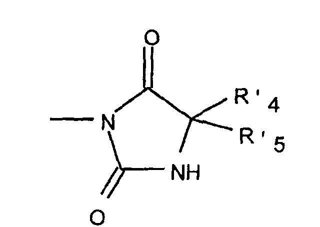 dans laquelle R'₄ et R'₅ ont les significations indiquées ci-dessus pour R₄ et R₅ dans lesquelles les éventuelles fonctions réactives sont éventuellement protégées,
   en faisant réagir sur le composé de formule (II) : 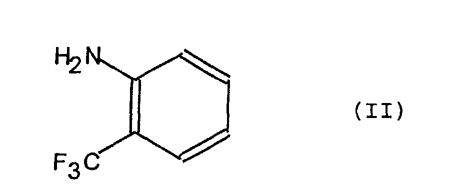 soit d'abord un composé de formule (III) : 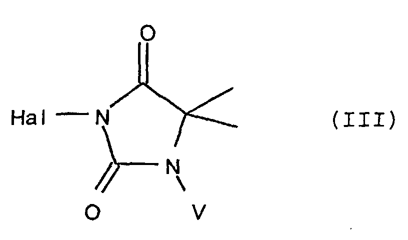 dans laquelle Hal représente un atome d'halogène et V représente un atome d'hydrogène ou un atome d'halogène, puis le composé de formule (B) : 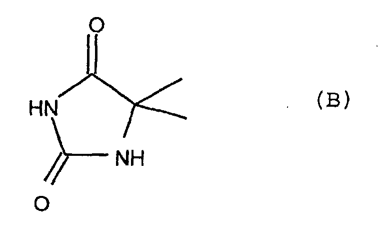 pour obtenir le produit de formule (A₁) : 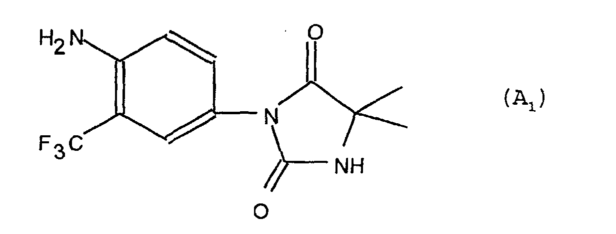 correspondant au produit de formule (A) dans laquelle W représente le radical diméthylhydantoïne : 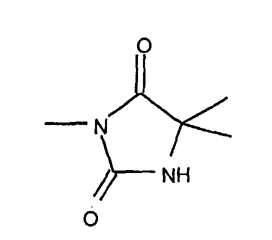 soit le N-bromosuccinimide dans le diméthylformamide, ou bien le composé de formule (III) telle que définie ci-dessus, pour obtenir le produit de formule (A₂) : 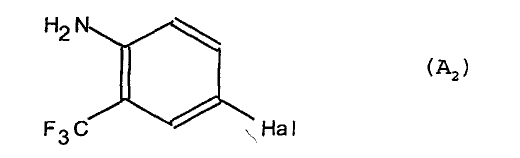 dans laquelle Hal représente un atome de brome ou un autre atome d'halogène, que l'on fait réagir avec un composé de formule (IV) : 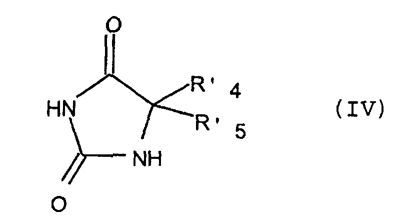 dans laquelle R'₄ et R'₅ ont les significations indiquées ci-dessus, pour obtenir le produit de formule (A₃) : 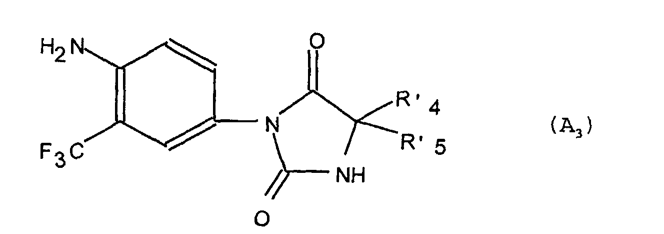 correspondant au produit de formule (A) dans laquelle W représente le radical : 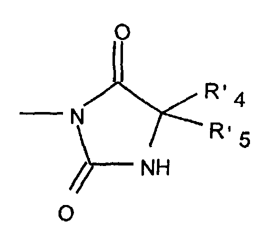 dans lequel R'₄ et R'₅ ont les significations indiquées ci-dessus,
b) si nécessaire et si désiré, on soumet le produit de formule (A) ainsi obtenu à l'une ou plusieurs des réactions suivantes, dans un ordre quelconque :
   i) une réaction de diazotation pour obtenir le produit de formule (V) : 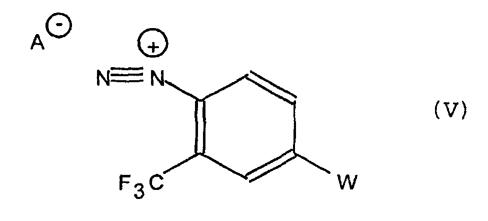 dans laquelle A^{⊖} représente un anion d'atome d'halogène ou de dérivé halogéné et W a la signification indiquée ci-dessus, que l'on soumet à une réaction d'halogénation pour obtenir le produit de formule (F₁) : 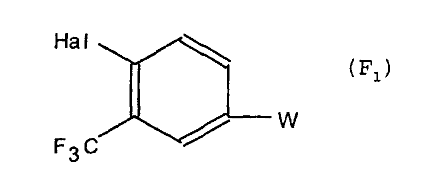 dans laquelle Hal et W ont les significations indiquées ci-dessus, que l'on peut soumettre à une réaction de substitution sur l'atome d'halogène par un dérivé métallique de formule (VI) :

      R'₁-M (VI)

      dans laquelle M représente un métal et R'₁ a la signification indiquée ci-dessus pour R₁, dans laquelle les éventuelles fonctions réactives sont éventuellement protégées,
      pour obtenir le produit de formule (F₂) : 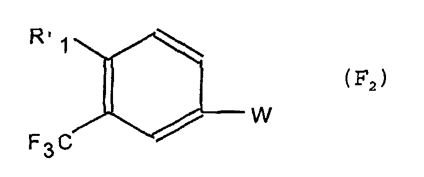 dans laquelle R'₁ et W ont les significations indiquées ci-dessus,
   ii) une réaction d'halogénation pour obtenir le produit de formule (F₃) : 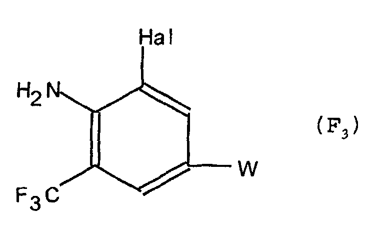 dans laquelle Hal représente un atome d'halogène et W a la signification indiquée ci-dessus, que l'on peut :
      soit soumettre à une réaction de substitution sur l'atome d'halogène, par un dérivé métallique de formule (VII) :

      R'₂-M (VII)

      dans laquelle M représente un métal et R'₂ a la signification indiquée ci-dessus pour R₂ dans laquelle les éventuelles fonctions réactives sont éventuellement protégées, pour obtenir le produit de formule (F₄) : 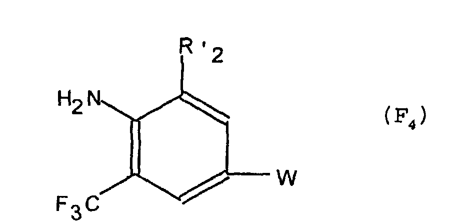
   dans laquelle R'₂ et W ont les significations indiquées ci-dessus,
   que l'on peut soumettre aux réactions successives, définies ci-dessus en i), de diazotation du radical amino, puis halogénation et enfin substitution par le composé de formule (VI) pour obtenir le produit de formule (F₅) : 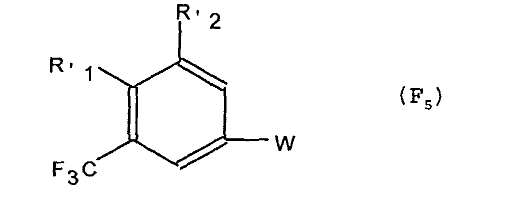 dans laquelle R'₁, R'₂ et W ont les significations indiquées ci-dessus,
   soit soumettre à une réaction diazotation-halogénation pour obtenir le produit de formule (F₆) : 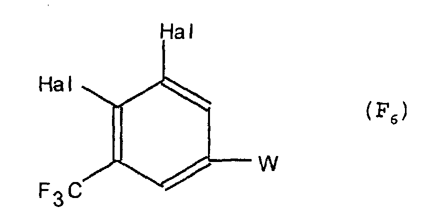 dans laquelle les deux atomes d'halogène représentés par Hal sont identiques ou différents et W a la signification indiquée ci-dessus, que l'on peut soumettre à une réaction de substitution sur les atomes d'halogène par le composé de formule (VI) ou (VII) telles que définies ci-dessus, pour obtenir le produit de formule (F₅) telle que définie ci-dessus dans laquelle R'₁ et R'₂ sont identiques,
c) on fait réagir, si nécessaire et si désiré, les produits de formules (F₁), (F₂), (F₃), (F₄), (F₅) et (F₆) lorsque W représente un atome d'halogène, avec le produit de formule (IV), telle que définie ci-dessus pour obtenir le produit de formule (I') : 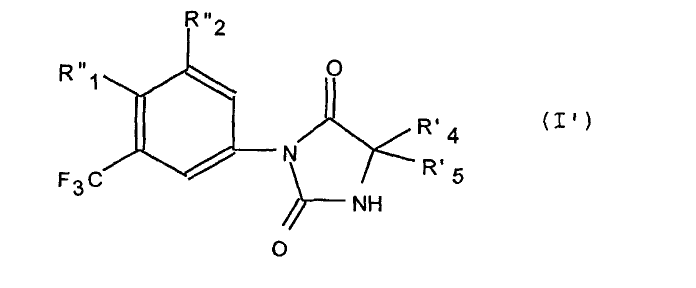 dans laquelle R"₁ et R"₂ sont tels que :
   soit R"₂ représente un atome d'hydrogène
   et R"₁ représente un atome d'halogène ou R'₁ tel que défini ci-dessus,
   soit R"₂ représente un atome d'halogène
   et R"₁ représente un radical amino ou un atome d'halogène, soit R"₂ représente R'₂ tel que défini ci-dessus
   et R"₁ représente un radical amino ou R'₁ tel que défini ci-dessus,
d) le cas échéant et si nécessaire, ou si désiré, on soumet les produits de formules (A₁), (A₃) et (I'), à l'une quelconque ou plusieurs des réactions suivantes, dans un ordre quelconque :
   a) réaction d'élimination des éventuels groupements protecteurs que peuvent porter, R"₁, R"₂, R'₄ et R'₅,
   b) réaction de transformation de groupement >C=O en groupement >C=S,
   c) action d'un réactif de formule Hal-R'₃ dans laquelle R'₃ a les valeurs de R₃ telle que définie ci-dessus, à l'exception de la valeur hydrogène et dans lesquelles les éventuelles fonctions réactives sont éventuellement protégées et Hal représente un atome d'halogène, pour obtenir des produits de formule (I) telle que définie à la revendication 1, puis, si désiré, action sur ces produits, d'un agent d'élimination des éventuels groupements protecteurs que peut porter R'₃ ou le cas échéant, action d'un agent d'estérification, d'amidification ou de salification,
   d) réaction de transformation de radical amino en radical nitro.

Pour la définition des substituants indiqués ci-dessus et dans ce qui suit, les définitions utilisées peuvent avoir les valeurs suivantes :

Par halogène, on entend bien entendu, les atomes de fluor, de chlore, de brome ou d'iode.

Le terme alkyle désigne un radical alkyle linéaire ou ramifié, ayant au plus 12 atomes de carbone, tel que par exemple les radicaux méthyle, éthyle, propyle, isopropyle, butyle, isobutyle, sec-butyle, tert-butyle, pentyle, isopentyle, sec-pentyle, tert-pentyle, néo-pentyle, hexyle, isohexyle, sec-hexyle, tert-hexyle, heptyle, octyle, décyle, undécyle, dodécyle.

On préfère les radicaux alkyle ayant au plus 6 atomes de carbone et notamment les radicaux méthyle, éthyle, propyle, isopropyle, pentyle ou hexyle.

Le terme alkényle désigne un radical alkényle linéaire ou ramifié, ayant au plus 12 atomes de carbone tel que par exemple les radicaux vinyle, allyle, 1-propényle, butényle, pentényle, hexényle.

Parmi les radicaux alkényle, on préfère ceux à 6 atomes de carbone tels que les radicaux allyle, propényle, butényle, pentényle ou hexényle.

Le terme alkynyle désigne un radical alkynyle linéaire ou ramifié, ayant au plus 12 atomes de carbone, tel que par exemple les radicaux éthynyle, propargyle, butynyle, pentynyle ou hexynyle.

Parmi les radicaux alkynyle, on préfère ceux à 4 atomes de carbone tels que le radical propargyle.

Le terme alcoxy désigne un radical linéaire ou ramifié, renfermant au plus 12 atomes de carbone et de préférence 6 tel que de préférence les radicaux méthoxy, éthoxy, propoxy ou isopropoxy, mais aussi butoxy linéaire, secondaire ou tertiaire, pentyloxy ou hexyloxy.

Par radical monoalkylamino, on entend de préférence les radicaux dans lesquels l'alkyle comprend au plus 4 atomes de carbone. On peut citer les radicaux méthylamino, éthylamino, propylamino ou butyl (linéaire ou ramifié) amino.

De même, par radical dialkylamino, on entend de préférence les radicaux dans lesquels l'alkyle comprend au plus 4 atomes de carbone. On peut citer par exemple les radicaux diméthylamino, diéthylamino, méthyléthylamino.

Le ou les radicaux carboxy des produits de formule (I) peuvent être salifiés, amidifiés ou estérifiés par les groupements divers connus de l'homme du métier.

Par carboxy estérifié on entend par exemple les radicaux alkyloxycarbonyle tels que par exemple les radicaux méthoxycarbonyle, éthoxycarbonyle, propoxycarbonyle, n-butyl-, tert-butyloxycarbonyle, ou encore benzyloxycarbonyle, ces radicaux alkyles pouvant être substitués par un ou plusieurs radicaux choisis par exemple parmi les atomes d'halogène, les radicaux hydroxyle, alcoxy, acyle, alcyloxy, alkylthio, amino ou aryle comme, par exemple, dans les groupements chlorométhyle, hydroxypropyle, propionyloxyméthyle, méthylthiométhyle, diméthylaminoéthyle, benzyle ou phénéthyle.

On peut citer des radicaux formés avec les restes esters facilement clivables tels que les radicaux méthoxyméthyle, éthoxyméthyle ; les radicaux acyloxyalkyle tels que pivaloyloxyméthyle, pivaloyloxyéthyle, acétoxyméthyle ou acétoxyéthyle ; les radicaux alkyloxycarbonyloxy alkyle tels que les radicaux méthoxycarbonyloxy méthyle ou éthyle, les radicaux isopropyloxycarbonyloxy méthyle ou éthyle.

Une liste de tels radicaux esters peut-être trouvée par exemple dans le brevet européen EP 0 034 536.

Par carboxy amidifié on entend les groupes du type -CON(R₆) (R₇) dans lesquels les radicaux R₆ et R₇ identiques ou différents représentent un atome d'hydrogène ou un radical alkyle ayant de 1 à 4 atomes de carbone tels que les radicaux méthyle, éthyle, propyle, isopropyle, butyle, isobutyle, sec-butyle ou tert-butyle.

Dans les groupes définis ci-dessus, -N(R₆)(R₇) représente ainsi le radical amino, ou un radical monoalkylamino ou diéthylamino tels que définis ci-dessus, mais peut également représenter un hétérocycle formé par R₆ et R₇ avec l'atome 'd'azote auquel ils sont attachés qui peut ou non comporter un hétéroatome supplémentaire. On peut citer les radicaux pyrrolyle, imidazolyle, indolyle, pipéridino, morpholino, pipérazinyle. On préfère les radicaux pipéridino, morpholino ou pipérazinyle éventuellement substitué sur le second atome d'azote, comme par exemple dans méthylpipérazinyle, fluorométhylpipérazinyle, éthylpipérazinyle, propylpipérazinyle, phénylpipérazinyle ou benzylpipérazinyle : dans ces deux derniers radicaux, les radicaux phényle et benzyle peuvent être substitués, comme par exemple dans chlorophényle ou trifluorophényle.

Par carboxy salifié on entend les sels formés par exemple avec un équivalent de sodium, de potassium, de lithium, de calcium, de magnésium ou d'ammonium. On peut également citer les sels formés avec les bases organiques telles que la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine.

On préfère le sel de sodium.

Par radical hydroxyle estérifié, éthérifié ou protégé, on entend respectivement les radicaux 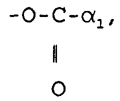 α₂-O-α₃ ou -O-P,
formés à partir d'un radical hydroxyle -OH, selon les méthodes usuelles connues de l'homme du métier et dans lesquels
P représente un groupement protecteur et α₁, α₂ et α₃ représentent notamment un radical alkyle, alkényle, alkynyle, aryle ou arylalkyle, ayant au plus 12 atomes de carbone et éventuellement substitués ainsi qu'il est défini ci-dessus.

Des exemples de groupement protecteur P, ainsi que la formation du radical hydroxyle protégé, sont donnés notamment dans le livre usuel de l'homme du métier : Protective Groups in Organic Synthesis, Theodora W. Greene, Harvard University, imprimé en 1981 par Wiley-Interscience Publishers, John Wiley & Sons.

Le groupement de protection du radical hydroxyle que peut représenter P, peut être choisi par exemple dans la liste suivante : formyle, acétyle, chloroacétyle, bromoacétyle, dichloroacétyle, trichloroacétyle, trifluoroacétyle, méthoxyacétyle, phénoxyacétyle, benzoyle, benzoylformyle, p-nitrobenzoyle. On peut citer également les groupements éthoxycarbonyle, méthoxycarbonyle, propoxycarbonyle, βββ-trichloroéthoxycarbonyle, benzyloxycarbonyle, tert-butoxycarbonyle, 1-cyclo propyléthoxycarbonyle, tétrahydropyrannyle, tétrahydrothiopyrannyle, méthoxytétrahydropyrannyle, trityle, benzyle, 4-méthoxybenzyle, benzhydryle, trichloroéthyle, 1-méthyl 1-méthoxyéthyle, phtaloyle, propionyle, butyryle, isobutyryle, valéryle, isovaléryle, oxalyle, succinyle et pivaloyle, phénylacétyle, phénylpropionyle, mésyle, chlorobenzoyle, para-nitrobenzoyle, para-tert-butylbenzoyle, caprylyle, acryloyle, méthylcarbamoyle, phénylcarbamoyle, naphtylcarbamoyle.
P peut notamment représenter le radical 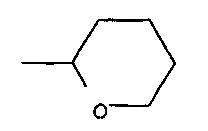 ou encore un dérivé du silicium tel que triméthylsilyle. - Le terme radical alkylthio désigne de préférence les radicaux dans lesquels le radical alkyle est tel que défini ci-dessus comme par exemple dans méthylthio, éthylthio, propylthio, isopropylthio, butylthio, sec-butylthio, tert-butylthio, isopentylthio ou isohexylthio ; le radical alkylthio est éventuellement substitué comme par exemple dans hydroxyméthylthio, aminoéthylthio, haloalkylthio tel que de préférence bromoéthylthio, trifluorométhylthio, trifluoroéthylthio ou encore pentafluoroéthylthio, arylalkylthio tel que par exemple benzylthio ou phénéthylthio.

Par radicaux alkyle, alkényle ou alkynyle éventuellement interrompus par un hétéroatome choisis parmi les atomes de soufre, d'oxygène ou d'azote, on entend les radicaux comprenant un ou plusieurs de ces atomes, identiques ou différents dans leur structure, ces hétéroatomes ne pouvant évidemment pas être situés à l'extrémité du radical. On peut citer par exemple les radicaux alkoxyalkyle tels que méthoxyméthyle, méthoxyéthyle ou propyloxypropyle, les radicaux alkoxyalkoxyalkyle tels que méthoxyéthoxyméthyle ou encore les radicaux alkylthioalkyle tels que par exemple propylthiopropyle, propylthioéthyle, méthylthiométhyle ou encore N-méthyl N-propylaminopropyle.

Dans tous ces radicaux, les atomes de soufre peuvent ne pas être oxydés comme dans les radicaux alkylthio, arylthio ou au contraire être oxydés pour donner les radicaux alkylsulfinyle, arylsulfinyle, alkylsulfonyle, ou arylsulfonyle : alkylsulfinyle et alkylsulfonyle désignent les radicaux dans lesquels le radical alkyle est choisi par exemple parmi les valeurs indiquées ci-dessus pour le radical alkyle tels que par exemple les radicaux méthylsulfinyle, éthylsulfinyle, méthylsulfonyle ou éthylsulfonyle, arylsulfinyle et arylsulfonyle désigne les radicaux arylthio, dans lesquels le radical aryle est choisi, par exemple, parmi les valeurs indiquées ci-dessus pour le radical aryle tels que par exemple les radicaux phényl-sulfinyle ou -sulfonyle, pyridyl-sulfinyle ou -sulfonyle, pyrimidyl-sulfinyle ou -sulfonyle, imidazolyl-sulfinyle ou -sulfonyle ou N-méthylimidazolyl-sulfinyle ou -sulfonyle.

Comme exemples particuliers de radicaux alkyle substitués par un ou plusieurs halogènes ou haloalkyle, on peut citer les radicaux monofluoro, chloro, bromo ou iodométhyle ou -éthyle, difluoro, dichloro ou dibromométhyle, trifluorométhyle ou pentafluoroéthyle.

Comme exemples particuliers de radicaux alcoxy substitués par un ou plusieurs halogènes ou haloalcoxy, on peut citer les radicaux bromoéthoxy, trifluorométhoxy, trifluoroéthoxy ou encore pentafluoroéthoxy.

Comme exemples particuliers de radicaux aryles ou aralkyles substitués, on peut citer ceux dans lesquels le radical phényle est substitué par un ou plusieurs radicaux choisis parmi les atomes d'iode, de chlore ou de brome, les radicaux méthoxy, trifluorométhyle, cyano ou amino.

Lorsque les produits de formule (I) telle que définie ci-dessus comportent un radical amino salifiable par un acide il est bien entendu que ces sels d'acides font également partie de l'invention.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, béta-éthanedisulfonique, les acides arylmonosulfoniques tels que l'acide benzènesulfonique et les acides aryldisulfoniques.

On peut citer plus particulièrement les sels formés avec les acides chlorhydrique ou méthanesulfonique par exemple.

La présente invention a plus particulièrement pour objet le procédé de préparation tel que défini ci-dessus des produits de formule (I) telle que définie ci-dessus dans laquelle :
R₁ et R₂, identiques ou différents sont tels que l'un représente un atome d'hydrogène ou un radical cyano et l'autre est choisi parmi les atomes d'halogène et les radicaux cyano et amino,
R₃ représente un atome d'hydrogène ou un radical alkyle éventuellement substitué par un radical hydroxyle éventuellement estérifié, éthérifié ou protégé,
R₄ et R₅, identiques ou différents, représentent un radical alkyle, linéaire ou ramifié renfermant au plus 6 atomes de carbone, éventuellement substitué par un ou plusieurs radicaux choisis parmi les radicaux hydroxyle éventuellement estérifié, éthérifié ou protégé et les atomes d'halogène et X et Y représentent un atome d'oxygène.

La présente invention a encore plus particulièrement pour objet le procédé de préparation tel que défini ci-dessus des produits suivants :
- 3-[4-amino-3-(trifluorométhyl) phényl] 5,5-diméthyl 2,4-imidazolidine dione,
- 5,5-diméthyl-3-(4-iodo-3-(trifluorométhyl) phényl) 2,4-imidazolidinedione,
- 4-(4,4-diméthyl-2,5-dioxo-3-(4-hydroxybutyl)-1-imidazolidinyl)-2-(trifluorométhyl)-benzonitrile,
- 4-(2,4-dioxo 1-(4-hydroxybutyl)-8-oxa-1,3-diazaspiro(4,5) décan-3-yl)-2-(trifluorométhyl) benzonitrile,
- 5,5-diméthyl-3-(4,5-dicyano-3-(trifluorométhyl) phényl)-2,4-imidazolidinedione,
ces produits étant sous toutes les formes isomères racémiques, énantiomères ou diastéréoisomères possibles, ainsi que leurs sels d'addition avec les acides minéraux et organiques ou avec les bases minérales et organiques pharmaceutiquement acceptables.

Pour mettre en oeuvre le procédé indiqué ci-dessus, on opère de préférence dans les conditions indiquées ci-après.

Pour obtenir le produit de formule (A₁), on préfère opérer en utilisant une demi-mole du composé de formule (III) pour une mole du composé de formule (II), dans du diméthylformamide ou de préférence du diméthylacétamide, à une température d'environ 0°C.

De façon préférée, le composé de formule (III) est la diméthyldibromohydantoïne utilisée en solution dans le diméthylacétamide et introduite sur l'orthotrifluorométhylaniline de formule (II) elle-même en solution dans la diméthylacétamide en maintenant la température à environ 0°C.

Le composé de formule (A₂) qui se forme in situ de façon intermédiaire, et que l'on n'isole pas, présente ainsi une bromation sélective qui se situe en position para du radical amino.

On ajoute alors in situ une demi-mole du composé B, soit la diméthylhydantoïne, de préférence en présence d'oxyde cuivreux à une température d'environ 155°C et obtient ainsi avec un remarquable rendement le produit de formule (A₁).

Pour isoler le produit de formule (A₂), la réaction du composé de formule (II) avec le composé de formule (III) peut être réalisée dans du diméthylacétamide, à une température de préférence à 0°C.

Le composé de formule (A₂) peut également être obtenu par bromation sélective par le N-bromosuccinimide sous forme solide ou en solution, en choisissant comme solvant le diméthylformamide ou le diméthylacétamide de préférence à l'emploi d'un solvant tel que l'eau, l'acétone ou d'autres solvants polaires utilisés usuellement.

De façon inattendue, on observe en effet dans ces conditions une remarquable sélectivité de la position de bromation.

On préfère également opérer dans ces conditions à une température de 0 à 20°C.

Le produit de formule (A₂) ainsi obtenu peut être soumis à une réaction avec un dérivé de l'hydantoïne, soit le composé de formule (IV), pour obtenir le produit de formule (A₃) ; on opère dans un solvant tel que le triglyme, le diméthylsulfoxyde, l'oxyde de diphényle, le diméthylformamide ou encore et de préférence le diméthylacétamide.

On opère de préférence en présence d'un catalyseur tel que le cuivre à l'état natif ou sous forme d'oxyde cuivreux ou cuivrique.

On préfère opérer dans du diméthylacétamide en présence d'oxyde cuivreux à une température de l'ordre de 165°C.

Le produit de formule (A) peut alors être soumis à une réaction de diazotation tel que par exemple par formation du chlorhydrate : on génère ainsi le sel de diazonium (N≡N^{⊕}-, Cl^{⊖}) en faisant agir du nitrite de sodium dans de l'acide chlorhydrique.

Le sel de diazonium ainsi obtenu peut être isolé si désiré, sous forme de sel de tétrafluoroborate (BF₄^{⊖}) insoluble dans l'eau par traitement par du tétrafluoroborate de sodium (NaBF₄).

Le sel de diazonium soit le produit de formule (V) obtenu peut alors être soumis à une réaction d'halogénation pour obtenir le produit de formule (F₁).

Cette halogénation peut être une bromuration par réaction, par exemple, de bromure de sodium ou de lithium dans un solvant tel que par exemple le mélange eau/chlorure de méthylène ou encore, et de préférence, une ioduration par action du iodure de sodium dans le mélange eau/chlorure de méthylène.

On peut également obtenir le produit de formule (F₁) dans lequel l'atome d'halogène est un fluor en chauffant le sel de diazonium isolé ci-dessus sous forme de tétrafluoroborate à une température de l'ordre de 60 à 80°C.

Le produit de formule (F₁) ainsi obtenu peut alors être soumis à une réaction de substitution sur l'atome d'halogène, qui est de préférence un atome d'iode, pour introduire le radical R'₁ et obtenir ainsi le produit de formule F₂. On opère dans un solvant tel que par exemple le diméthylformamide. Dans les composés de formules (VI) et (VII), M représente un métal tel que le cuivre ou le nickel, ou encore le palladium en particulier pour introduire un radical acétylénique. Les composés de formules (VI) et (VII) peuvent ainsi notamment être le cyanure de cuivre ou encore le trifluorométhyle cuprate (CF₃Cu) obtenu par réaction du triméthyl(trifluorométhyl) silane avec du fluorure de potassium et de l'iodure de cuivre dans le diméthylformamide.

La réaction d'halogénation du produit de formule (A) pour donner le produit de formule (F₃) peut être effectuée dans les conditions usuelles telles que par exemple par bromuration par le N-bromo succinimide, dans un solvant tel que par exemple le diméthylformamide à une température de l'ordre de 20 à 30°C : l'atome d'halogène est ainsi introduit en position ortho du radical amino.

Le produit de formule (F₃) pour donner le produit de formule (F₄) peut être soumis à une réaction de substitution de l'atome d'halogène par le radical R'₂ selon les conditions usuelles connues de l'homme du métier et notamment telles que définies ci-dessus, pour introduire le radical R'₁ sur le produit de formule (F₁).

L'amine de formule (F₄) ainsi obtenue peut être transformée en sel de diazonium ensuite halogéné et enfin substitué sur l'atome d'halogène par le radical R'₁ dans les mêmes conditions que celles décrites ci-dessus, pour donner ainsi le produit de formule (F₅).

La réaction d'halogénation du produit de formule (F₃) en produit de formule (F₆) peut être réalisée selon les conditions usuelles notamment par formation du sel de diazonium sur le radical amino puis halogénation dans les conditions définies ci-dessus.

Le produit de formule (F₆) peut être à son tour substitué sur les deux atomes d'halogène notamment par un même radical cyano par exemple par action de cyanure de cuivre dans du diméthylformamide.

Les produits de formule (F₁), (F₂), (F₃), (F₄), (F₅) ou (F₆) peuvent être soumis à l'action du produit de formule (IV) pour donner le produit correspondant de formule (I) dans les conditions définies ci-dessus de réaction du produit de formule (A₂) avec le produit de formule (IV) pour donner le produit de formule (I').

Les produits de formules (A₁), (A₃) et (I') ainsi obtenus peuvent alors si nécessaire et si désiré, être soumis à une réaction de substitution par un dérivé halogéné de formule R'₃-Hal dans laquelle R'₃ peut notamment représenter un dérivé acylé tel que notamment le composé ZO-alk-Hal dans lequel alk représente un radical alkyle, Z un radical acyle tel que notamment le radical acétyle ou encore un radical silyle et Hal représente un atome d'halogène tel que de préférence un atome de brome, d'iode ou de chlore de préférence au fluor.

On opère dans un solvant tel que par exemple et notamment le diméthylformamide ou le diméthylacétamide en présence d'une base forte telle que la soude, l'hydrure de sodium ou de potassium. On peut opérer par réaction de transfert de phase en présence de sels d'ammonium quaternaires tels que le tert-butyl ammonium.

On obtient ainsi notamment les produits de formule (I) dans laquelle R₃ représente un radical alkyle substitué par un radical hydroxyle, libre, estérifié, éthérifié ou protégé tel que acylé ou silylé.

Les éventuelles fonctions réactives que peuvent porter ou représenter R"₁, R"₂, R'₃, R'₄ ou R'₅ et qui sont éventuellement protégées, peuvent être notamment les fonctions hydroxy ou amino. On utilise pour protéger ces fonctions des groupements protecteurs usuels. On peut citer par exemples les groupements protecteurs suivants du radical amino : tert-butyle, tert-amyle, trichloroacétyle, chloroacétyle, benzhydryle, trityle, formyle, benzyloxycarbonyle.

Comme groupement protecteur du radical hydroxy on peut citer les radicaux tels que formyle, chloroacétyle, tétrahydropyrannyle, triméthylsilyle, tert-butyl diméthylsilyle.

Il est bien entendu que la liste ci-dessus n'est pas limitative et que d'autres groupements protecteurs, par exemple connus dans la chimie des peptides peuvent être utilisés. Une liste de tels groupements protecteurs se trouve par exemple dans le brevet français BF 2.499.995.

Les réactions éventuelles d'élimination des groupements protecteurs sont effectuées comme indiqué dans ledit brevet BF 2.499.995. Le mode préféré d'élimination est l'hydrolyse acide à l'aide des acides choisis parmi les acides chlorhydrique, benzène sulfonique ou para toluène sulfonique, formique ou trifluoroacétique. On préfère l'acide chlorhydrique.
- L'estérification éventuelle des produits, dans lesquels R'₃ comporte un radical OH libre est effectuée dans des conditions classiques. On peut utiliser par exemple un acide ou un dérivé fonctionnel, par exemple un anhydride tel que l'anhydride acétique en présence d'une base telle que la pyridine.

L'estérification ou la salification éventuelle des produits, dans lesquels R'₃ comporte un groupement COOH est effectuée dans les conditions classiques connues de l'homme du métier.
- L'amidification éventuelle des produits, dans lesquels R'₃ comporte un radical COOH est effectuée dans des conditions classiques. On peut utiliser une amine primaire ou secondaire sur un dérivé fonctionnel de l'acide par exemple un anhydride symétrique ou mixte.

La réaction de transformation du ou des groupements >C=O en groupement >C=S est effectuée à l'aide du réactif dit de Lawesson de formule : 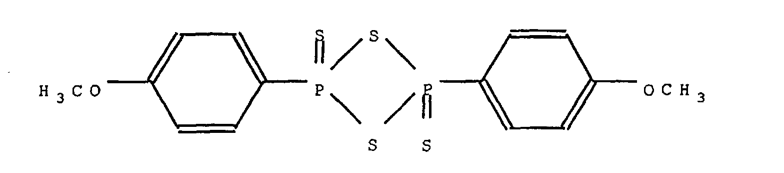 qui est un produit commercialisé par exemple par la firme FLUKA et dont l'utilisation est décrite par exemple dans la publication : Bull. Soc. Chim. Belg. vol 87, N° 3, (1987) p. 229.

Lorsque l'on veut transformer deux fonctions >C=O en deux fonctions >C=S on opère en présence d'un excès de réactif de Lawesson. Il en est de même lorsque l'on part d'une molécule comportant une fonction >C=S et une fonction >C=O et que l'on veut transformer ladite fonction >C=O en fonction >C=S.

Par contre lorsque l'on part d'une molécule comportant deux fonctions >C=O et que l'on veut obtenir un produit ne comportant qu'une seule fonction >C=S. On opère en présence d'un déficit de réactif de Lawesson. On obtient alors en général un mélange de trois produits : chacun des deux produits comportant une fonction >C=O et une fonction >C=S et le produit comportant deux fonctions >C=S. Ces produits peuvent être ensuite séparés par les méthodes usuelles telles que la chromatographie.

La réaction de transformation du radical amino en radical nitro peut être réalisée dans les conditions usuelles connues de l'homme du métier, telles que notamment celles décrites dans les références suivantes :
- Emmons W.D., J. Am. Chem. Soc. 1957, 79, 5528,
- Holmes R R and Bayer R p, J. Am. Chem. Soc. 1960, 82, 3454.

Un procédé de préparation de certains produits de formule (I) telle que définie ci-dessus est décrit dans le brevet français n° 2 693 461.

La présente invention a tout particulièrement pour objet un procédé de préparation des produits de formule (I) telle que définie ci-dessus, caractérisé en ce que pour obtenir le produit de formule (A₁) à partir des produits de formules (II), (III) et B, telles que définies ci-dessus, on opère dans un solvant choisi parmi le diméthylsulfoxyde, le triglyme, le diméthylacétamide ou le diméthylformamide et de préférence le diméthylacétamide.

La présente invention a plus particulièrement pour objet un procédé de préparation des produits de formule (I) telle que définie ci-dessus, caractérisé en ce que le composé de formule (III) est le dérivé dibromé de formule : 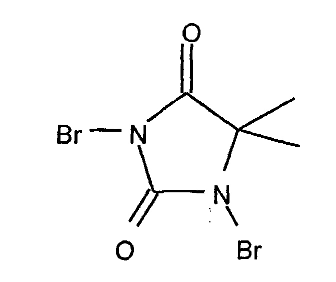 et que l'on utilise une demi-mole de ce composé et une demi-mole du composé de formule (B) pour une mole du composé de formule (II).

La présente invention a encore plus particulièrement pour objet un procédé de préparation des produits de formule (I) telle que définie ci-dessus, caractérisé en ce que la réaction est réalisée à une température de 130°C à 160°C et de préférence à 155°C.

Les produits de départ de formule (II), (III), (B), (IV), (VI) et (VII) sur lesquels s'exerce le procédé, objet de l'invention, pour l'obtention des produits de formule (I), sont connus et disponibles dans le commerce ou peuvent être préparés selon des méthodes connues de l'homme de métier.

Les produits de formule (IV) qui sont des dérivés de l'hydantoïne sont largement utilisés et cités dans la littérature comme par exemple dans les articles suivants :
- J. Pharm. Pharmacol., 67, Vol. 19(4), p. 209-16 (1967)
- J. Chem. Soc., 74, (2), p. 219-21 (1972)
- Khim. Farm. Zh., 67, Vol. 1 (5) p. 51-2
- Brevet allemand 2.217.914
- Brevet européen 0.091.596
- J. Chem. Soc. Perkin. Trans. 1, 74 (2) p. 48, p. 219-21.

La présente invention a encore pour objet à titre de produits industriels nouveaux, les produits suivants :
- 3-[4-amino 3-(trifluorométhyl) phényl]-5,5-diméthyl 2,4-imidazolidine dione,
- 5,5-diméthyl 3-(4-iodo 3-(trifluorométhyl) phényl)-2,4-imidazolidinedione,
- 5,5-diméthyl 3-(4,5-dicyano 3-(trifluorométhyl) phényl) 2,4-imidazolidinedione.

Les exemples donnés ci-après illustrent l'invention.

### EXEMPLE 1 : 3-[4-amino 3-(trifluorométhyl) phényl] 5,5-diméthyl 2,4-imidazolidine dione

On introduit à 20°±2°C 100 g d'O-trifluorométhylaniline puis ajoute en maintenant la même température 100 ml de diméthylacétamide. On refroidit alors sous agitation à 0°C±2°C et ajoute en 30 minutes environ une solution de 88,8 g de dibromodiméthylhydantoïne et 100 ml de diméthylacétamide en maintenant à 0°C±2°C. On maintient 30 minutes sous agitation, puis porte à 20°C±2°C et ajoute 40 g de diméthylhydantoïne puis 50 g d'oxyde de cuivre. On chauffe au reflux pendant environ 18 heures puis refroidit à 20°C±2°C, agite 30 minutes puis filtre, essore et lave par 4 x 25 ml de diméthylacétamide. On verse alors sous agitation en 1 heure à 20°C±2°C, 300 ml d'ammoniaque 22°Bé pure et 300 ml d'eau déminéralisée, agite 1 heure à 20°C±2°C puis refroidit à 0°C±5°C et maintient encore 1 heure sous agitation. Puis on essore, lave à 20°C±2°C, par 100 ml d'ammoniaque 22°Bé pure puis par 4 x 100 ml d'eau déminéralisée et sèche. On obtient ainsi 155,8 g de produit attendu.
Analyses : IR CHCl₃ (cm⁻¹)
- NH/NH₂ =C-NH₂: 3510
- =C-NH: 3449
- =C-NH₂: 3429
- 〉=O: 1781-1719
- Aromatiques + NH₂ déf.: 1637-1585-1516-1511

### EXEMPLE 2 : 5,5-diméthyl-3-(4-iodo3-(trifluorométhyl) phényl)-2,4-imidazolidine dione

On introduit à 20°C±2°C, 140 g du produit de l'exemple 1 et 210 ml d'eau déminéralisée, met sous agitation et ajoute en 5 minutes environ, 210 ml d'acide chlorhydrique pur 22°Bé. On maintient 30 minutes à 35°-40°C sous agitation puis refroidit à 0°±5°C sous agitation. On ajoute alors 28 ml de chlorure de méthylène puis en 30 minutes environ, à 0°±5°C, une solution de 43,7 g de nitrite de sodium dans 70 ml d'eau déminéralisée. On maintient encore 1 h à 0°±5°C sous agitation, ajoute en 45 minutes une solution de 87,7 g d'iodure de sodium dans 140 ml d'eau déminéralisée. On maintient encore 1 heure sous agitation et ajoute 700 ml de chlorure de méthylène. On agite 15 mn à 0°±5°C, ajoute en 1 fois 28 g de métabisulfite de sodium et agite encore 30 minutes en laissant la température revenir vers 20°C. On verse, décante la phase organique, réextrait la phase aqueuse avec 280 ml de chlorure de méthylène, puis lave les phases organiques par 3 x 140 ml de solution aqueuse saturée en ClNa. Les phases chlorométhyléniques jointes sont séchées. On filtre et lave par 3 x 70 ml de chlorure de méthylène et obtient 184,5 g de produit attendu (cristaux blancs) F = 164-165°C.

### EXEMPLE 3 : 4-(4,4-diméthyl-2,5-dioxo-1-imidazolidinyl)-2-(trifluorométhyl)-benzonitrile

On introduit 184 g du produit de l'exemple 2 à 20°±2°C et ajoute sous agitation 66,15 g de cyanure de cuivre et 420 ml de diméthylformamide puis chauffe tout en distillant le chlorure de méthylène jusqu'à obtenir une température de 130°C dans le milieu réactionnel et maintient alors 5 heures sous agitation à cette température. On refroidit à 20°±2°C sous agitation, maintient 1 heure dans ces conditions puis essore et lave par 3 x 0,3 vol de diméthylformamide. On ajoute alors sur le mélange agité à 20°±2°C, 700 ml d'ammoniaque pure 22°Bé et 700 ml d'eau déminéralisée. On agite 1 heure à 20°±2°C puis refroidit à 0°±5°C, maintient 1 heure sous agitation à 0°±5°C, essore et lave par 2 x 140 ml d'ammoniaque pure 22°Bé à 20°±2°C puis par 4 x 140 ml d'eau déminéralisée puis sèche. On purifie en ajoutant 1105 ml d'acétate d'éthyle puis porte au reflux sous agitation et ajoute alors 12,3 g de noir acticarbone CX. On maintient 30 minutes sous agitation au reflux, puis filtre, lave par 3 x 61 ml d'acétate d'éthyle bouillant, concentre sous agitation, refroidit sous agitation à 0°±2°C et maintient dans ces conditions pendant 2 heures. On essore et lave par 3 x 37 ml d'acétate d'éthyle à 0°±2°C et sèche. On obtient ainsi 103,7 g de produit attendu (poudre beige clair).
F° = 210°C
Analyses : IR nujol (cm⁻¹)
- Région OH/NH: max 3340
- -C≡N: 2245
- 〉=O: 1789-1720
- Aromatiques: 1612-1575-1505

### EXEMPLE 4 : 4- (4,4-diméthyl-2,5-dioxo-3-(4-hydroxybutyl) 1-imidazolidinyl)-2-(trifluorométhyl)-benzonitrile

On introduit à 20°/22°C, 300 ml de diméthylformamide et 100 g du produit de l'exemple 3 et maintient sous agitation, à cette température environ 5 minutes puis ajoute 98,5 g de 4-bromobutyle acétate puis 20 g de soude et maintient sous agitation et sous azote à +20°/+22°C pendant environ 22 heures. On ajoute en maintenant sous agitation, à cette température, 20 g de soude puis en 5 minutes environ 400 ml de méthanol et maintient ainsi pendant 1 heure. On introduit sous agitation en laissant évoluer la température, 500 ml d'eau déminéralisée à +20°C, puis place sous agitation, ajoute 500 ml d'eau déminéralisée à +20°C et maintient pendant 1 heure à 25°/30°C sous agitation, puis refroidit sous agitation, à +0°/+5°C, et maintient pendant 2 heures. On essore, lave avec 4 x 100 ml d'eau déminéralisée et sèche. On purifie par ajout à 20°/22°C de 696 ml de chlorure de méthylène et lave avec 3 x 232 ml d'eau déminéralisée puis sèche, rajoute 5,8 g de noir supra, maintient sous agitation, à 20°±2°C, pendant 2 heures puis filtre et rince avec 2x116 ml de chlorure de méthylène. On concentre sous agitation, ajoute 116 ml d'éthanol denat. toluène à 20°C puis 174 ml d'eau déminéralisée. On refroidit sous agitation, à 20°/22°C, maintient sous agitation, pendant 2 heures à cette température puis refroidit à 0°±2°C et maintient 1 heure dans ces conditions. On essore, lave avec 2 x 58 ml d'éthanol à 50 % eau à 0°/+2°C et sèche. On obtient ainsi 111,5 g de produit attendu (poudre blanche). F = 102°C.

### EXEMPLE 5 : 4-(4,4-diméthyl 2,5-dioxo 1-imidazolidinyl) 2-(trifluorométhyl) benzonitrile

### Stade 1 : para-bromo ortho-trifluorométhyl aniline

### 1ère méthode :

On introduit 100 g d'ortho trifluorométhylaniline et 200 ml de diméthylacétamide et refroidit à 0°±2°C. On ajoute en 30 minutes à 0°±2°C 88,8 g de dibromo-diméthyl hydantoïne, maintient la température à 0°±2°C. et agite à 0°±2°C pendant 15 minutes. Puis on laisse la température remonter à 20°C et verse sur 200 ml d'eau déminéralisée à 20°±2°C. On agite pendant 15 minutes, ajoute 400 ml d'éther isopropylique, décante la phase aqueuse et lave la phase organique par 2 x 100 ml d'eau déminéralisée, les phases aqueuses sont réextraites par 100 ml d'éther isopropylique et les phases organiques réunies sont séchées, filtrées et lavées par 2 x 20 ml d'éther isopropylique. On concentre à une température de 30-40°C et obtient ainsi 149 g de produit attendu (huile brun-orangé).

### 2ème méthode :

On introduit 100 g d'ortho trifluorométhylaniline et 200 ml de diméthylacétamide et ajoute en 30 minutes environ à 20°±2°C 107,3 g de N-bromo succinimide en poudre. On maintient la température à 20°±2°C et agite sous azote à 20°±2°C pendant 15 minutes, puis verse sur 200 ml d'eau déminéralisée à 20°±2°C, agite 15 minutes et ajoute 400 ml d'éther isopropylique. On décante la phase aqueuse, lave la phase organique par 2 x 100 ml d'eau déminéralisée, les phases aqueuses sont réextraites par 100 ml d'éther isopropylique et les phases organiques réunies sont séchées. On filtre, lave par 2 x 20 ml d'éther isopropylique, concentre et obtient ainsi 149 g de produit attendu.
Analyses : IR sur CHCl₃ (cm⁻¹)
- =C-NH₂: 3520-3430
- NH₂ déf. + aromatiques: 1634-1610-1581-1492

### Stade 2 : fluoroborate de p-bromo o-trifluorométhyl diazonium

On introduit à 20°C, 120 g du produit obtenu au stade 1 ci-dessus et 240 ml d'eau déminéralisée, puis en 15 minutes environ, en laissant évoluer la température à 35-40°C, 375 ml d'acide chlorhydrique concentré 22°Bé. On agite 30 minutes en laissant la température redescendre à 20°C, refroidit à 0°C±2°C, et introduit en 30 minutes environ, en maintenant la température à 0°C±2°C, la solution de 240 ml d'eau déminéralisée et 72,5 g de nitrite de sodium et agite en maintenant la température à 0°C±2°C pendant 1 heure. On ajoute à cette température 140 g de tétrafluoroborate de sodium, maintient sous agitation à 0°C±2°C, pendant 1 heure et filtre, rince par 2 x 50 ml d'eau déminéralisée glacée et obtient ainsi 194,14 g de produit attendu.

### Stade 3 : p-bromo o-trifluorométhyl benzonitrile

On introduit à 20°C, 13,5 g de cyanure de cuivre et 400 ml d'eau déminéralisée, maintient la température à +20°C±2°C, ajoute en 5 minutes environ la solution de 41,6 g de cyanure de sodium et 100 ml d'eau déminéralisée, refroidit à 0°C±2°C et introduit en maintenant cette température en 10 minutes environ 194 g de sel de diazonium obtenu au stade 2 ci-dessus. On maintient sous agitation, à 0°C±2°C, pendant 1 heure puis laisse remonter la température à 20°C et ajoute 50 ml d'ammoniaque concentré et 1 litre de chlorure de méthylène. On décante, lave, sèche, concentre, reprend dans 160 ml d'heptane, filtre, sèche et purifie par chromatographie sur silice avec pour éluant heptane-acétate d'éthyle (9-1) et obtient ainsi 86 g de produit attendu (cristaux blancs).
F = 30°C.
Analyses : IR CHCl₃ (cm⁻¹)
- -C≡N: ∼ 2240
- Aromatiques: 1598-1570-1488

### Stade 4 : 4-(4,4-diméthyl-2,5-dioxo-1-imidazolidinyl)-2-(trifluorométhyl)-benzonitrile

On introduit à 20°C, 110 g du produit obtenu au stade 3 ci-dessus et 275 ml de diméthyl acétamide. On rajoute à 20°C sous agitation 31,7 g d'oxyde de cuivre Cu₂O et 67,7 g de diméthyl hydantoïne. On chauffe pendant 5 heures environ à 165°C, laisse refroidir à 20°C, puis filtre et rince avec 3 x 55 ml de diméthyl acétamide. On prépare une solution de 550 ml d'ammoniaque concentré 22°Bé et 550 ml d'eau glacée et l'introduit en 15 minutes environ à 0°C et laisse environ 1 heure à 0°C, puis essore, lave par 110 ml de solution aqueuse 50 % ammoniaque puis par 4 x 110 ml d'eau déminéralisée. On sèche et purifie en ajoute 125 ml de toluène et 125 ml d'acétonitrile puis chauffe à 80°C, pendant 1 heure, et laisse refroidir. On agite ensuite 1 heure à 0°C, filtre, essore et lave par 2 x 25 ml de solution glacée (acétonitrile/toluène (1:1)). On sèche et obtient ainsi 104,4 g de produit attendu.
F = 210°C.

### EXEMPLE 6 (ne fait pas partie de l'invention) : 4-(4,4-diméthyl-2,5-dioxo-1-imidazolidinyl)-2-(trifluorométhyl)-benzonitrile

### Stade 1 : 3-(trifluorométhyl)-4-cyano chloro benzène

On introduit à 20°C 100 g de 2-trifluorométhyl-4-chloro iodobenzène, 200 ml de diméthylformamide et 58,7 g de cyanure de cuivre, chauffe pendant 3 heures à 140°C, laisse refroidir à 20°C, puis verse sur 600 ml d'eau déminéralisée glacée. On filtre, rince avec 3 x 200 ml d'éther isopropylique, décante la phase aqueuse et la réextrait avec 3 x 200 ml d'éther isopropylique. Les phases organiques sont réunies et lavées par 200 ml d'eau déminéralisée et séchées. On obtient ainsi 66,64 g de produit attendu.
Analyses : IR sur CHCl₃ (cm⁻¹)
- C≡N: ~ 2238
- Aromatiques: 1601-1570

### Stade 2 : 4-(4,4-diméthyl-2,5-dioxo-1-imidazolidinyl)-2-(trifluorométhyl)-benzonitrile

On introduit 4,47 g du produit obtenu au stade 1 ci-dessus, 11,2 ml de triglyme, 2,78 g de 5,5-diméthyl hydantoïne et 1,34 g d'oxyde cuivreux puis agite la suspension et porte à 215°C pendant 4 heures. Puis on ramène à température ambiante, filtre, lave par 4,5 ml de triglyme et agite sans dépasser 25°C, 4,5 ml d'ammoniaque concentrée 22°Bé, 26 ml d'eau et 4,5 ml de toluène. On agite 15 minutes à 20°C puis refroidit à -10°C, agite 1 heure, essore, lave par 2,2 ml de toluène puis 4,5 ml d'eau et sèche. On obtient ainsi 1,98 g de produit attendu (cristaux bruns). F = 210°C.
Analyses : IR nujol (cm⁻¹)
- Absorption OH/NH: ∼ 3340
- C≡N: ∼ 2240
- 〉=O: 1788-1721
- Aromatiques: 1610-1572-1504

### EXEMPLE 7 : 5,5-diméthyl-3-(4,5-dicyano-3-(trifluorométhyl) phényl) 2,4-imidazolidinedione

### Stade 1 : 3-(5,5-diméthyl-2,4-imidazolidine)-2-amino 3-(trifluorométhyl) bromo benzène

On introduit à 20±2°C, 20 g du produit de l'exemple 1 et 40 ml de diméthylacétamide, refroidit à +10°C±2°C et sous agitation et azote, ajoute en 30 minutes environ à 10°C±2°C, 12,5 g de N-bromo succinimide en poudre. On maintient la température à 10°C±2°C, agite pendant 15 minutes, laisse remonter à 20°C puis agite 1 heure. On verse sur 200 ml de chlorure de méthylène, introduit 100 ml d'eau déminéralisée à 20°C±2°C, décante, lave la phase organique par 2 x 50 ml d'eau déminéralisée à 20°C±2°C, sèche et concentre. On obtient ainsi 22 g de produit attendu.

### Stade 2 : 4-(5,5-diméthyl-2,4-imidazolidinedione)-2-bromo 5-(trifluorométhyl) iodo benzène

On introduit à 20°C, 20 g du produit obtenu au stade 1 ci-dessus et 30 ml d'eau déminéralisée et introduit en 15 mn, en laissant évoluer la température à 35-40°C, 30 ml d'acide chlorhydrique concentré 22°Bé. On agite 30 minutes en laissant la température redescendre à 20°C, refroidit à 0°C±2°C et introduit en 30 minutes en maintenant cette température, la solution de 12 ml d'eau déminéralisée et 4,9 g de nitrite de sodium. On agite en maintenant cette température pendant 1 heure et rajoute, sous agitation 100 ml de chlorure de méthylène puis en 30 minutes, la solution de 9,83 g d'iodure de sodium et 10 ml d'eau déminéralisée et maintient, sous agitation à 0°C±2°C pendant 1 heure, puis laisse remonter la température à 10°C. On ajoute ensuite 4 g de métabisulfite de sodium, décante, lave la phase chlorométhylénique à l'eau, sèche et concentre. On obtient ainsi 18,5 g de produit attendu.
Analyses : CHCl₃ (cm⁻¹)
- =C-NH: 3446
- 〉=O: 1790-1730
- Aromatiques: 1597-1560

### Stade 3 : 5,5-diméthyl-3-(4,5-dicyano 3-(trifluorométhyl) phényl)-2,4-imidazolidinedione

On introduit à 20°C, 13 g du produit obtenu au stade 2 ci-dessus, 26 ml de diméthylformamide, 2,7 g de cyanure de cuivre et 1,47 g de cyanure de sodium, et chauffe pendant 20 heures à 150°C. Puis on laisse refroidir à 20°C, verse sur le mélange 50 ml d'eau déminéralisée et 50 ml d'ammoniaque pur 22°, filtre, rince avec 3 x 50 ml de chlorure de méthylène, décante la phase aqueuse et la réextrait avec 3 x 50 ml de chlorure de méthylène. Les phases organiques sont réunies et lavées par 50 ml d'eau déminéralisée et séchées. La phase chlorométhylénique est agitée à 20°C, pendant une heure avec 1,5 g de noir acticarbone et le chlorure de méthylène est évaporé et remplacé par 30 ml d'éther isopropylique. On essore à 20°C, lave par 3 x 10 ml d'éther isopropylique et sèche. On purifie par chromatographie sur silice avec pour éluant chlorure de méthylène-acétate d'éthyle (95-5), puis par dissolution dans de l'isopropanol à reflux, filtre, rince à l'isopropanol, concentre, glace 1 heure, essore et sèche. On obtient ainsi 3,1 g de produit attendu (cristaux blancs). F = 159-160°C.
Analyses : IR
- OH/NH: 3403-3388
- C≡N: 2236
- 〉=O: 1776-1738-1729
- Aromatiques: 1606-1575-1502.

### EXEMPLE 8 : 4-(2,4-dioxo 8-oxa 1,3-diaza spiro[4,5]decan 3-yl) 2-trifluorométhyl) aminobenzyle

On agite 18 heures à 150°-155°C, un mélange de 7 g de para-bromo-orthotrifluorométhylaniline obtenue au stade 1 de l'exemple 5, 15 ml de diméthylacétamide, 2,33 g d'oxyde cuivreux et 6 g de 5[spiro(4-pyran)] 2,4-imidazolidine dione (dont la préparation est donnée ci-après). On refroidit à 20-22°C, filtre, lave avec 2 fois 7 ml de diméthylacétamide et coule dans 200 ml d'eau. On agite 1 heure à température ambiante, essore et lave avec un mélange d'eau et d'ammoniaque à 20 % (50/50) puis à l'eau. Après séchage à 40°C, on recueille 9,1 g du produit recherché.

### PREPARATION DE : 5[spiro(4-pyran)] 2,4-imidazolidine dione utilisé au départ de l'exemple 8.

On chauffe 4 heures à 45-50°C, 5 g de tétrahydro-4h-pyran-4-one, 25 ml d'eau déminéralisée, 25 ml d'éthanol, 7,2 g de cyanure de potassium et 57 g de carbonate d'ammonium. On concentre sous pression réduite jusqu'à sec. On reprend l'extrait sec avec 50 ml d'eau, essore, lave et sèche à 40°C. On obtient 7,2 g de produit attendu.
Spectre RMN (DMSO)
1,47-1,84 : les CH₂-C ; 3,59-3,81 : les CH₂O ; 8,57-10,67 : les NH-C=O.

### EXEMPLE 9 : 4-(2,4-dioxo 8-oxa 1,3-diaza spiro[4,5]decan 3-yl) 2-trifluorométhyl) iodobenzyle

On opère comme à l'exemple 2, à partir de 8 g du produit obtenu à l'exemple 8 en utilisant 10 ml d'acide chlorhydrique 22°Bé, 2,18 g de nitrite de sodium et 5,5 g d'iodure de sodium. On recueille ainsi 8,9 g du produit recherché.

### EXEMPLE 10 : 4-(2,4-dioxo 8-oxa 1,3-diaza spiro[4,5]decan 3-yl) 2-trifluorométhyl) benzonitrile

On opère comme à l'exemple 3, en utilisant 3,2 g de cyanure de cuivre. Après recristallisation dans l'isopropanol, on recueille 1,8 g du produit recherché.
Spectre RMN : CDCl₃
1,78 (m), 2,55 (m) : C-CH₂ ; 3,70 (m), 4,13 (m) : CHO ; 6,21 (s) : CONH ; 7,95 (m), 8,11 (m) : H aromatiques.

### EXEMPLE 11 : 4-(2,4-dioxo 1-(4-hydroxybutyl) 8-oxa 1,3-diaza spiro[4,5]decan 3-yl) 2-trifluorométhyl) benzonitrile

On introduit 55 g d'hydrure de sodium à 50 % dans l'huile et ajoute en 25 minutes, 340 mg du produit obtenu à l'exemple 10 en solution dans 25 ml de diméthylformamide, 20 minutes après la fin du dégagement d'hydrogène, on ajoute 0,41 g de 4-iodobutoxy triméthylsilane et agite 18 heures à température ambiante. On verse sur 10 ml d'eau, extrait avec de l'éther éthylique, lave à l'eau puis à l'eau salée, sèche et ajoute 10 ml de méthanol et 1 ml d'acide chlorhydrique 2N, agite 30 minutes et coule dans 20 ml d'eau saturée en ClNa, extrait avec du chloroforme, sèche, évapore à sec et chromatographie le résidu sur silice en éluant avec le mélange chlorure de méthylène-acétone (8-2). On obtient 369 mg du produit recherché.
Spectre I.R. (CHCl₃) cm⁻¹
- OH: 3626-3485
- C≡N: 2235
- C=O: 1775-1721
- aromatiques: 1615-1602-1575-1505.

## Revendications

1. Procédé de préparation des produits de formule (I) : 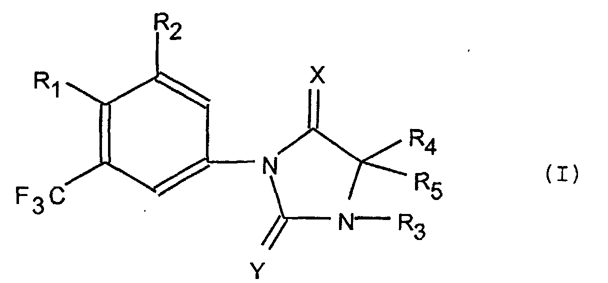 dans laquelle :
R₁ et R₂, identiques ou différents, sont choisis parmi l'atome d'hydrogène, les atomes d'halogène et les radicaux alkyle, alkényle, alkynyle, cyano, trifluorométhyle, amino, monoalkylamino et dialkylamino,
R₃ représente un atome d'hydrogène, un radical alkyle, éventuellement interrompu par un ou plusieurs atomes d'oxygène ou de soufre, un radical phényle ou pyridyle, ces radicaux étant éventuellement substitués par un ou plusieurs radicaux choisis parmi les atomes d'halogène, le radical phényle, hydroxyle éventuellement estérifié, éthérifié ou protégé, alkoxy, cyano, trifluorométhyle, hydroxyalkyle, carboxy libre, estérifié, amidifié ou salifié, amino, mono ou dialkylamino, l'atome d'azote du radical pyridyle étant éventuellement oxydé,
R₄ et R₅
soit identiques ou différents, représentent un radical alkyle, éventuellement substitué par un ou plusieurs radicaux choisis parmi les radicaux hydroxyle éventuellement estérifié, éthérifié ou protégé, les atomes d'halogène et les radicaux alkylthio et phénylthio eux-mêmes éventuellement substitués par un ou plusieurs radicaux choisis parmi les atomes d'halogène et le radical hydroxyle,
soit forment ensemble le radical : 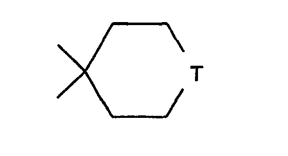 dans lequel T représente un atome d'oxygène ou de soufre, X et Y, identiques ou différents, représentent un atome d'oxygène ou de soufre,
lesdits produits de formule (I) étant sous toutes les formes isomères racémiques, énantiomères ou diastéréoisomères possibles, ainsi que les sels d'addition avec les acides minéraux et organiques ou avec les bases minérales et organiques desdits produits de formule (I), **caractérisé en ce que** :
a) on prépare un produit de formule (A) : 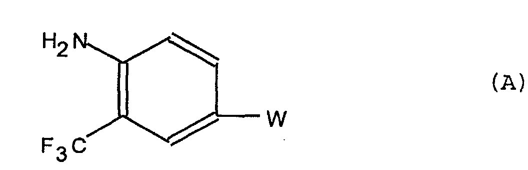 dans laquelle W représente un atome d'halogène ou un dérivé de l'hydantoïne de formule : 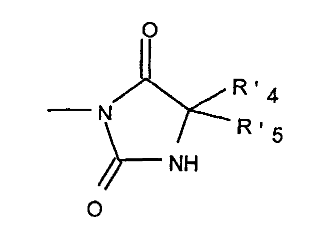 dans laquelle R'₄ et R'₅ ont les significations indiquées ci-dessus pour R₄ et R₅ dans lesquelles les éventuelles fonctions réactives sont éventuellement protégées,
en faisant réagir sur le composé de formule (II) : 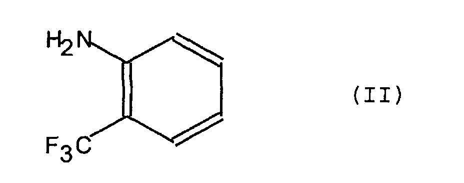 soit d'abord un composé de formule (III) : 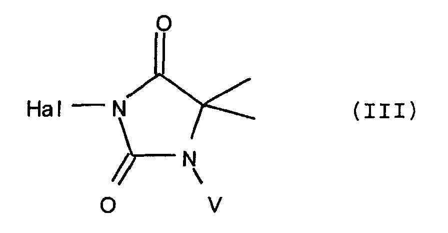 dans laquelle Hal représente un atome d'halogène et V représente un atome d'hydrogène ou un atome d'halogène, puis le composé de formule (B) : 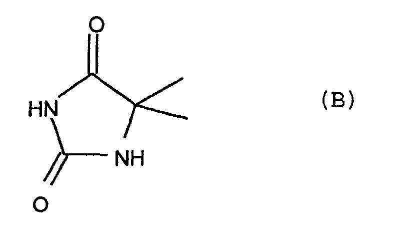 pour obtenir le produit de formule (A₁) : 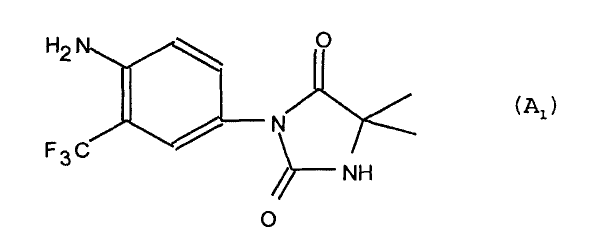 correspondant au produit de formule (A) dans laquelle W représente le radical diméthylhydantoïne : 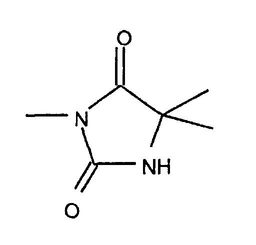 soit le N-bromosuccinimide dans le diméthylformamide, ou bien le composé de formule (III) telle que définie ci-dessus, pour obtenir le produit de formule (A₂) : 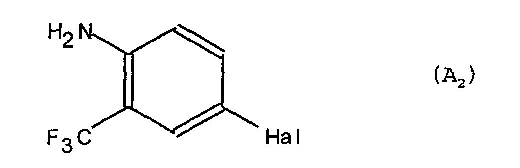 dans laquelle Hal représente un atome de brome ou un autre atome d'halogène, que l'on fait réagir avec un composé de formule (IV) : 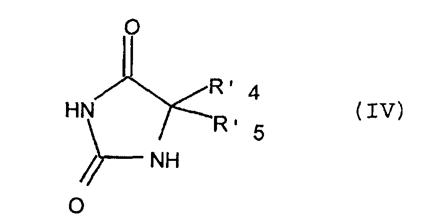 dans laquelle R'₄ et R'₅ ont les significations indiquées ci-dessus, pour obtenir le produit de formule (A₃) : 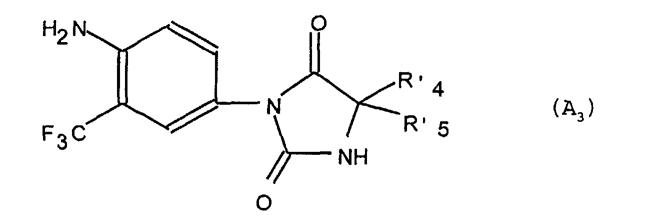 correspondant au produit de formule (A) dans laquelle W représente le radical : 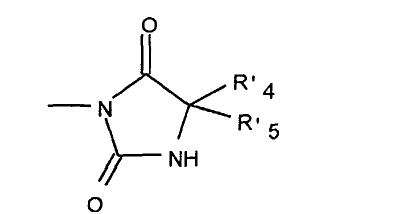 dans lequel R'₄ et R'₅ ont les significations indiquées ci-dessus,
b) si nécessaire et si désiré, on soumet le produit de formule (A) ainsi obtenu à l'une ou plusieurs des réactions suivantes, dans un ordre quelconque :
**i)** une réaction de diazotation pour obtenir le produit de formule (V) : 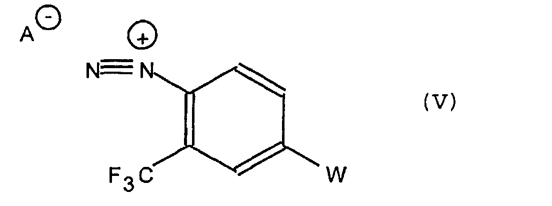 dans laquelle A^{⊖} représente un anion d'atome d'halogène ou de dérivé halogéné et W a la signification indiquée ci-dessus,
que l'on soumet à une réaction d'halogénation pour obtenir le produit de formule (F₁) : 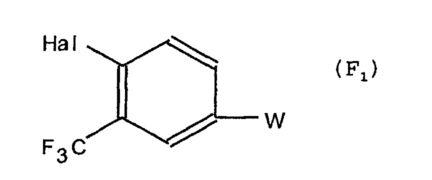 dans laquelle Hal et W ont les significations indiquées ci-dessus, que l'on peut soumettre à une réaction de substitution sur l'atome d'halogène par un dérivé métallique de formule (VI) :
R'₁-M (VI)
dans laquelle M représente un métal et R'₁ a la signification indiquée ci-dessus pour R₁, dans laquelle les éventuelles fonctions réactives sont éventuellement protégées,
pour obtenir le produit de formule (F₂) : 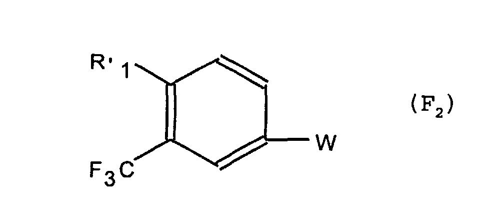 dans laquelle R'₁ et W ont les significations indiquées ci-dessus,
**ii)** une réaction d'halogénation pour obtenir le produit de formule (F₃) : 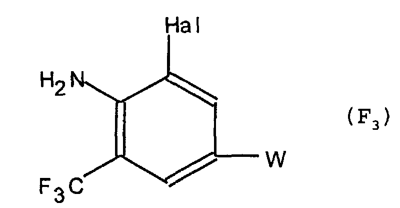 dans laquelle Hal représente un atome d'halogène et W a la signification indiquée ci-dessus, que l'on peut :
soit soumettre à une réaction de substitution sur l'atome d'halogène, par un dérivé métallique de formule (VII) :
R'₂-M (VII)
dans laquelle M représente un métal et R'₂ a la signification indiquée ci-dessus pour R₂ dans laquelle les éventuelles fonctions réactives sont éventuellement protégées, pour obtenir le produit de formule (F₄) : 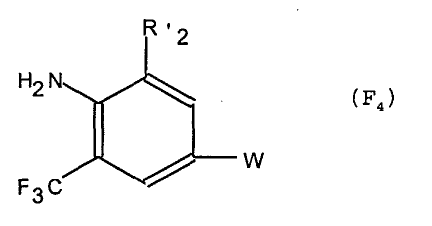 dans laquelle R'₂ et W ont les significations indiquées ci-dessus,
que l'on peut soumettre aux réactions successives, définies ci-dessus en i), de diazotation du radical amino, puis halogénation et enfin substitution par le composé de formule (VI) pour obtenir le produit de formule (F₅) : 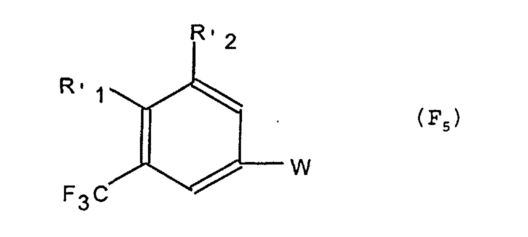 dans laquelle R'₁, R'₂ et W ont les significations indiquées ci-dessus,
soit soumettre à une réaction diazotation-halogénation pour obtenir le produit de formule (F₆) : 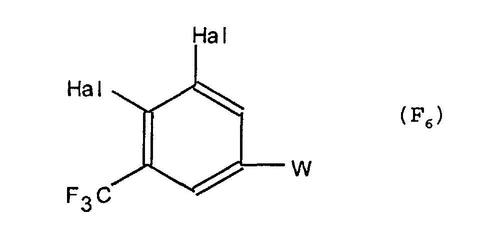 dans laquelle les deux atomes d'halogène représentés par Hal sont identiques ou différents et W a la signification indiquée ci-dessus, que l'on peut soumettre à une réaction de substitution sur les atomes d'halogène par le composé de formule (VI) ou (VII) telles que définies ci-dessus, pour obtenir le produit de formule (F₅) telle que définie ci-dessus dans laquelle R'₁ et R'₂ sont identiques,
c) on fait réagir, si nécessaire et si désiré, les produits de formules (F₁), (F₂), (F₃), (F₄), (F₅) et (F₆) lorsque W représente un atome d'halogène, avec le produit de formule (IV), telle que définie ci-dessus pour obtenir le produit de formule (I') : 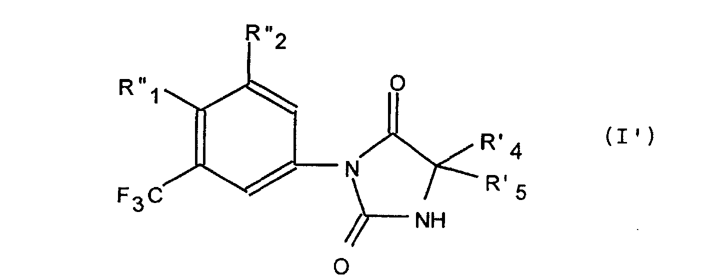 dans laquelle R"₁ et R"₂ sont tels que :
soit R"₂ représente un atome d'hydrogène
et R"₁ représente un atome d'halogène ou R'₁ tel que défini ci-dessus,
soit R"₂ représente un atome d'halogène
et R"₁ représente un radical amino ou un atome d'halogène,
soit R"₂ représente R'₂ tel que défini ci-dessus
et R"₁ représente un radical amino ou R'₁ tel que défini ci-dessus,
d) le cas échéant et si nécessaire, ou si désiré, on soumet les produits de formules (A₁), (A₃) et (I'), à l'une quelconque ou plusieurs des réactions suivantes, dans un ordre quelconque :
a) réaction d'élimination des éventuels groupements protecteurs que peuvent porter, R"₁, R"₂, R'₄ et R'₅,
b) réaction de transformation de groupement >C=O en groupement >C=S,
c) action d'un réactif de formule Hal-R'₃ dans laquelle R'₃ a les valeurs de R₃ telle que définie ci-dessus, à l'exception de la valeur hydrogène et dans lesquelles les éventuelles fonctions réactives sont éventuellement protégées et Hal représente un atome d'halogène, pour obtenir des produits de formule (I) telle que définie à la revendication 1, puis, si désiré, action sur ces produits, d'un agent d'élimination des éventuels groupements protecteurs que peut porter R'₃ ou le cas échéant, action d'un agent d'estérification, d'amidification ou de salification,
d) réaction de transformation de radical amino en radical nitro.

2. Procédé de préparation tel que défini à la revendication 1, des produits de formule (I), telle que définie à la revendication 1, dans laquelle R₁ et R₂, identiques ou différents sont tels que l'un représente un atome d'hydrogène ou un radical cyano et l'autre est choisi parmi les atomes d'halogène et les radicaux cyano et amino,
R₃ représente un atome d'hydrogène ou un radical alkyle éventuellement substitué par un radical hydroxyle éventuellement estérifié, éthérifié ou protégé,
R₄ et R₅, identiques ou différents, représentent un radical alkyle, linéaire ou ramifié renfermant au plus 6 atomes de carbone, éventuellement substitué par un ou plusieurs radicaux choisis parmi les radicaux hydroxyle éventuellement estérifié, éthérifié ou protégé et les atomes d'halogène et X et Y représentent un atome d'oxygène.

3. Procédé de préparation tel que défini à la revendication 1, des produits suivants :
- 3-[4-amino-3-(trifluorométhyl) phényl] 5,5-diméthyl 2,4-imidazolidine dione,
- 5,5-diméthyl-3-(4-iodo-3-(trifluorométhyl) phényl) 2,4-imidazolidinedione,
- 4-(4,4-diméthyl-2,5-dioxo-3-(4-hydroxybutyl)-1-imidazolidinyl)-2-(trifluorométhyl)-benzonitrile,
- 4-(2,4-dioxo 1-(4-hydroxybutyl)-8-oxa-1,3-diazaspiro(4,5) décan-3-yl)-2-(trifluorométhyl) benzonitrile,
- 5,5-diméthyl-3-(4,5-dicyano-3-(trifluorométhyl) phényl)-2,4-imidazolidinedione,
ces produits étant sous toutes les formes isomères racémiques, énantiomères ou diastéréoisomères possibles, ainsi que leurs sels d'addition avec les acides minéraux et organiques ou avec les bases minérales et organiques pharmaceutiquement acceptables.

4. Procédé de préparation des produits de formule (I) telle que définie aux revendications 1 à 3, **caractérisé en ce que** pour obtenir le produit de formule (A₁) à partir des produits de formules (II), (III) et B, telles que définies à la revendication 1, on opère dans un solvant choisi parmi le diméthylsulfoxyde, le triglyme, le diméthylacétamide ou le diméthylformamide et de préférence le diméthylacétamide.

5. Procédé de préparation des produits de formule (I) telle que définie à la revendication 4, **caractérisé en ce que** le composé de formule (III) est le dérivé dibromé de formule : 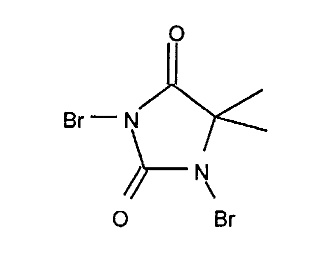 et que l'on utilise une demi-mole de ce composé et une demi-mole du composé de formule (B) pour une mole du composé de formule (II).

6. Procédé de préparation des produits de formule (I) telle que définie aux revendications 4 et 5, **caractérisé en ce que** la réaction est réalisée à une température de 130°C à 160°C et de préférence à 155°C.

## Claims

1. Process for the preparation of the products of formula (I): 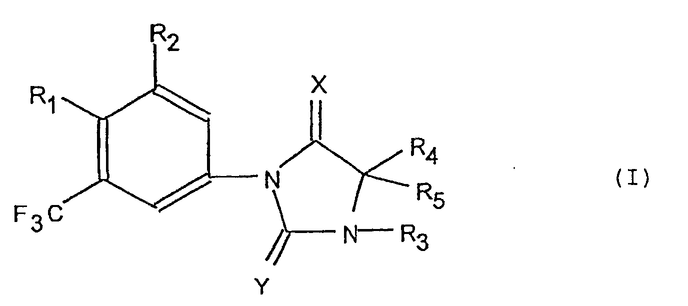 in which:
R₁ and R₂, identical or different, are chosen from the hydrogen atom, the halogen atoms and the alkyl, alkenyl, alkynyl, cyano, trifluoromethyl, amino, monoalkylamino and dialkylamino radicals,
R₃ represents a hydrogen atom, an alkyl radical, optionally interrupted by one or more oxygen or sulphur atoms, a phenyl or pyridyl radical, these radicals being optionally substituted by one or more radicals chosen from the halogen atoms, the phenyl, the optionally esterified, etherified or protected hydroxyl, alkoxy, cyano, trifluoromethyl, hydroxyalkyl, free, esterified, amidified or salified carboxy, amino, mono- or dialkylamino radical, the nitrogen atom of the pyridyl radical being optionally oxidized,
R₄ and R₅
either identical or different, represent an alkyl radical, optionally substituted by one or more radicals chosen from the optionally esterified, etherified or protected hydroxyl radicals, the halogen atoms and the alkylthio and phenylthio radicals themselves optionally substituted by one or more radicals chosen from the halogen atoms and the hydroxyl radical,
or together form the: 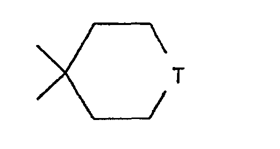 radical in which T represents an oxygen or sulphur atom,
X and Y, identical or different, represent an oxygen or sulphur atom,
said products of formula (I) being in all possible racemic, enantiomeric and diastereoisomeric isomer forms,
as well as the addition salts with mineral and organic acids or with mineral and organic bases of said products of formula (I), **characterized in that**:
a) a product of formula (A): 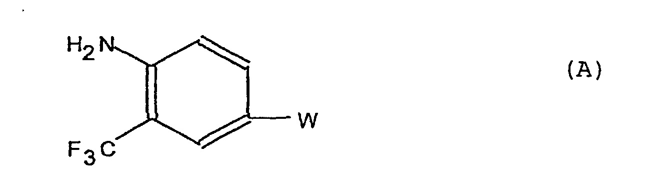 is prepared, in which W represents a halogen atom or a hydantoin derivative of formula: 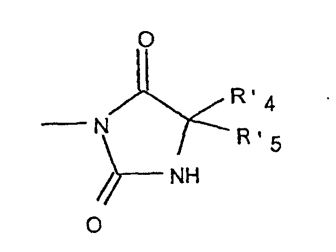 in which R'₄ and R'₅ have the meanings indicated above for R₄ and R₅ in which the optional reactive functions are optionally protected,
by reacting with the compound of formula (II): 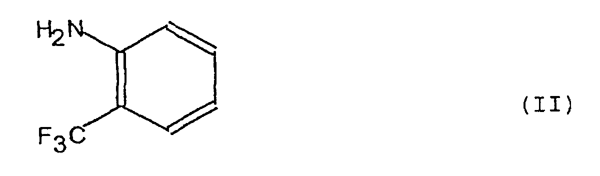 either firstly a compound of formula (III): 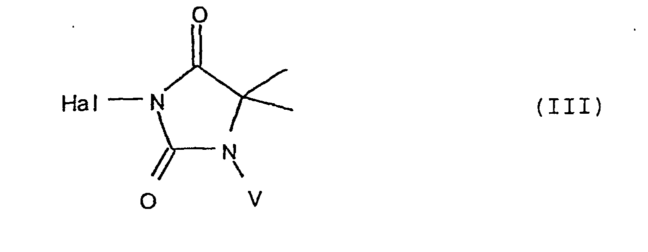 in which Hal represents a halogen atom and V represents a hydrogen atom or a halogen atom, then the compound of formula (B): 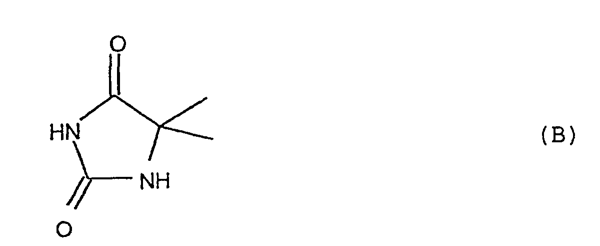 in order to obtain the product of formula (A₁): 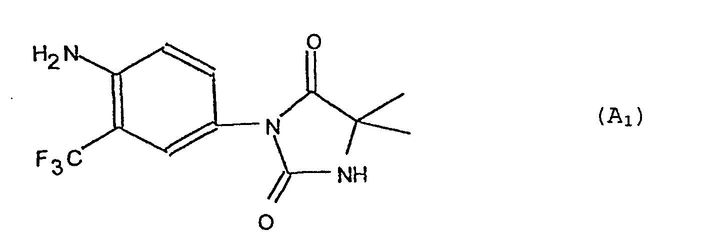 corresponding to the product of formula (A) in which W represents the dimethylhydantoin radical: 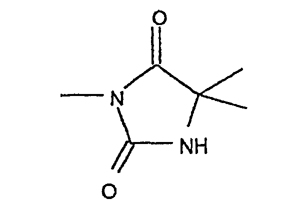 or N-bromosuccinimide in dimethylformamide, or the compound of formula (III) as defined above, in order to obtain the product of formula (A₂): 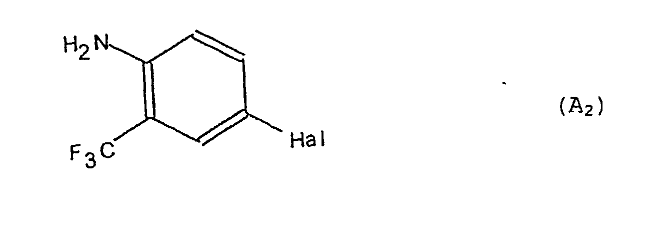 in which Hal represents a bromine atom or another halogen atom, which is reacted with a compound of formula (IV): 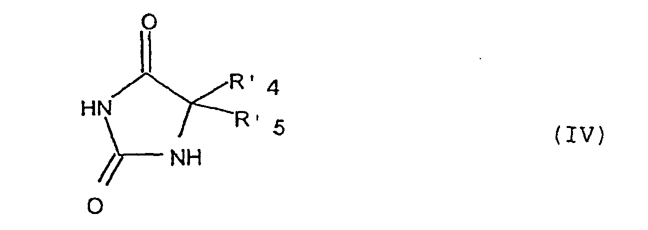 in which R'₄ and R'₅ have the meanings indicated above,
in order to obtain the product of formula (A₃): 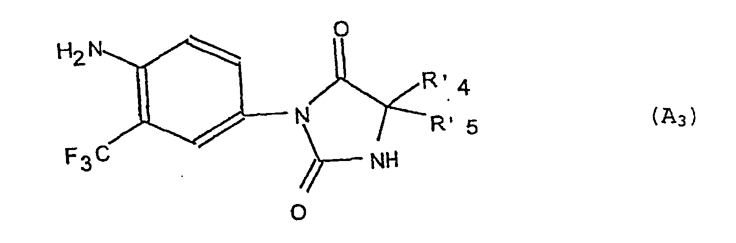 corresponding to the product of formula (A) in which W represents the: 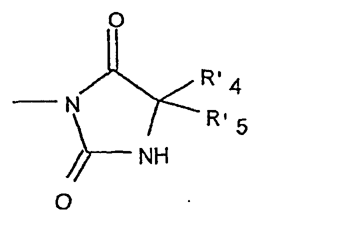 radical in which R'₄ and R'₅ have the meanings indicated above,
b) if necessary and if desired, the product of formula (A) thus obtained is subjected to one or more of the following reactions, in any order:
**i)** a diazotization reaction in order to obtain the product of formula (V): 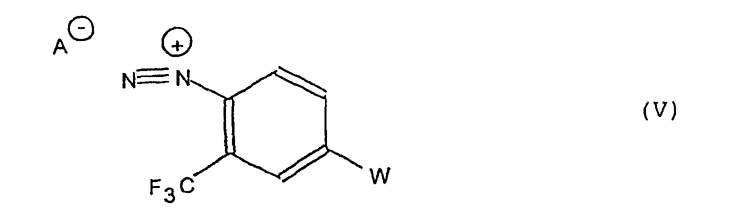 in which A⁻ represents an anion of a halogen atom or halogenated derivative and W has the meaning indicated above, which is subjected to a halogenation reaction in order to obtain the product of formula (F₁): 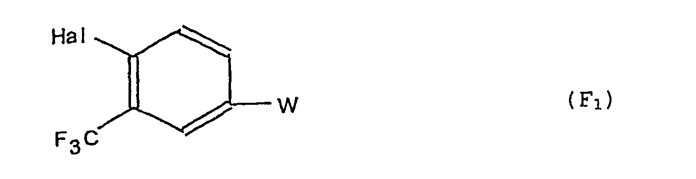 in which Hal and W have the meanings indicated above, which can be subjected to a substitution reaction on the halogen atom by a metallic derivative of formula (VI):
R'₁-M (VI)
in which M represents a metal and R'₁ has the meaning indicated above for R₁, in which the optional reactive functions are optionally protected,
in order to obtain the product of formula (F₂): 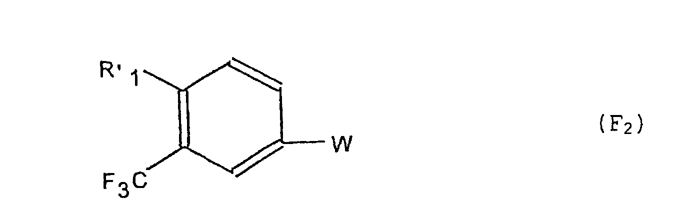 in which R'₁ and W have the meanings indicated above,
**ii)** a halogenation reaction in order to obtain the product of formula (F₃): 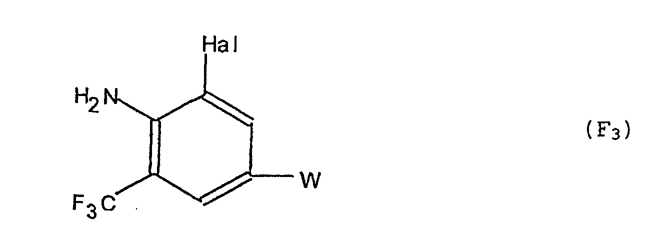 in which Hal represents a halogen atom and W has the meaning indicated above, which can:
either be subjected to a substitution reaction on the halogen atom, by a metallic derivative of formula (VII):
R'₂-M (VII)
in which M represents a metal and R'₂ has the meaning indicated above for R₂ in which the optional reactive functions are optionally protected, in order to obtain the product of formula (F₄): 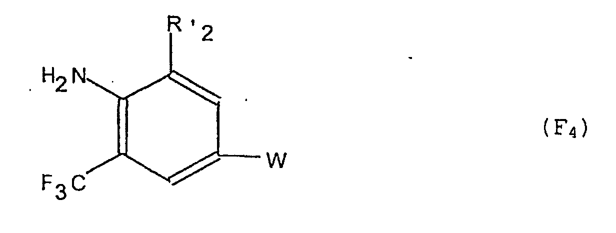 in which R'₂ and W have the meanings indicated above,
which can be subjected to successive reactions, defined above in i), of diazotization of the amino radical, then halogenation and finally substitution by the compound of formula (VI) in order to obtain the product of formula (F₅): 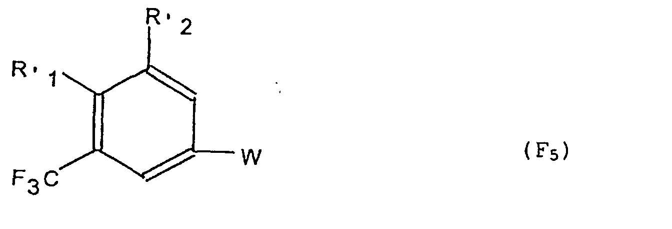 in which R'₁, R'₂ and W have the meanings indicated above,
or subjected to a diazotization-halogenation reaction in order to obtain the product of formula (F₆): 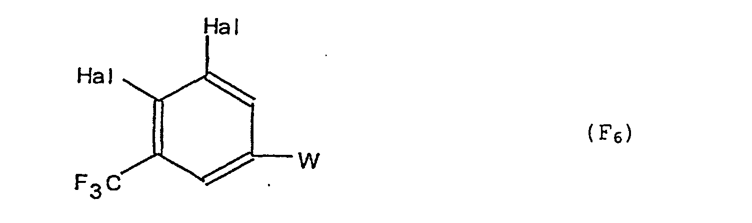 in which the two halogen atoms represented by Hal are identical or different and W has the meaning indicated above, which can be subjected to a substitution reaction on the halogen atoms by the compound of formula (VI) or (VII) as defined above, in order to obtain the product of formula (F₅) as defined above in which R'₁ and R'₂ are identical,
c) if necessary and if desired, the products of formulae (F₁), (F₂), (F₃), (F₄), (F₅) and (F₆) when W represents a halogen atom, are reacted with the product of formula (IV), as defined above in order to obtain the product of formula (I'): 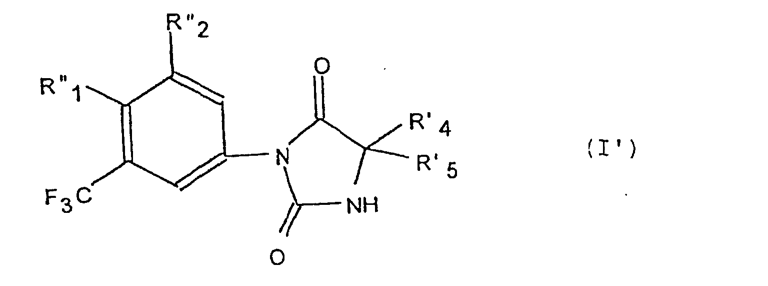 in which R"₁ and R"₂ are such that:
either R"₂ represents a hydrogen atom
and R"₁ represents a halogen atom or R'₁ as defined above,
or R"₂ represents a halogen atom
and R"₁ represents an amino radical or a halogen atom,
or R"₂ represents R'₂ as defined above
and R"₁ represents an amino radical or R'₁ as defined above,
d) if appropriate and if necessary, or if desired, the products of formulae (A₁), (A₃) and (I'), are subjected to any one or more of the following reactions, in any order:
a) elimination reaction of the optional protective groups that can be carried by R"₁, R"₂, R'₄ and R'₅,
b) conversion reaction of the >C=O group to a >C=S group,
c) the action of a reagent of formula Hal-R'₃ in which R'₃ has the values of R₃ as defined above, with the exception of the hydrogen value and in which the optional reactive functions are optionally protected and Hal represents a halogen atom, in order to obtain products of formula (I) as defined in claim 1, then, if desired, the action on these products, of an elimination agent of the optional protective groups which can be carried by R'₃ or if appropriate, the action of an esterification, amidification or salification agent,
d) conversion reaction of the the amino radical to a nitro radical.

2. Process for the preparation, as defined in claim 1, of the products of formula (I), as defined in claim 1, in which R₁ and R₂, identical or different, are such that one represents a hydrogen atom or a cyano radical and the other is chosen from the halogen atoms and the cyano and amino radicals,
R₃ represents a hydrogen atom or an alkyl radical optionally substituted by an optionally esterified, etherified or protected hydroxyl radical
R₄ and R₅, identical or different, represent a linear or branched alkyl radical containing at most 6 carbon atoms, optionally substituted by one or more radicals chosen from the optionally esterified, etherified or protected hydroxyl radicals and the halogen atoms and X and Y represent an oxygen atom.

3. Process for the preparation as defined in claim 1, of the following products:
- 3-[4-amino-3-(trifluoromethyl)phenyl] 5,5-dimethyl 2,4-imidazolidine dione,
- 5,5-dimethyl-3-(4-iodo-3-(trifluoromethyl) phenyl) 2,4-imidazolidinedione,
- 4-(4,4-dimethyl-2,5-dioxo-3-(4-hydroxybutyl)-1-imidazolidinyl)-2-(trifluoromethyl)-benzonitrile,
- 4-(2,4-dioxo 1-(4-hydroxybutyl)-8-oxa-1,3-diazaspiro(4,5) decan-3-yl)-2-(trifluoromethyl) benzonitrile,
- 5,5-dimethyl-3-(4,5-dicyano-3-(trifluoromethyl) phenyl)-2,4-imidazolidinedione,
these products being in all possible racemic, enantiomeric and diastereoisomeric isomer forms, as well as their addition salts with pharmaceutically acceptable mineral and organic acids or mineral and organic bases.

4. Process for the preparation of the products of formula (I) as defined in claims 1 to 3, **characterized in that** in order to obtain the product of formula (A₁) starting from the products of formulae (II), (III) and B, as defined in claim 1, the operation is carried out in a solvent chosen from dimethylsulphoxide, triglyme, dimethylacetamide or dimethylformamide and preferably dimethylacetamide.

5. Process for the preparation of the products of formula (I) as defined in claim 4, **characterized in that** the compound of formula (III) is the dibrominated derivative of formula: 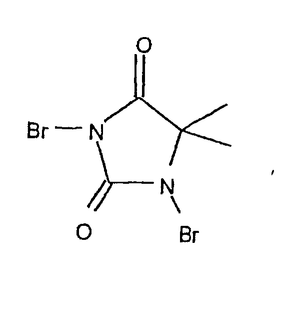 and that a demi-mole of this compound and a demi-mole of the compound of formula (B) is used for one mole of the compound of formula (II).

6. Process for the preparation of the products of formula (I) as defined in claims 4 and 5, **characterized in that** the reaction is carried out at a temperature of 130°C to 160°C and preferably at 155°C.

## Patentansprüche

1. Verfahren zur Herstellung der Produkte der Formel (I): 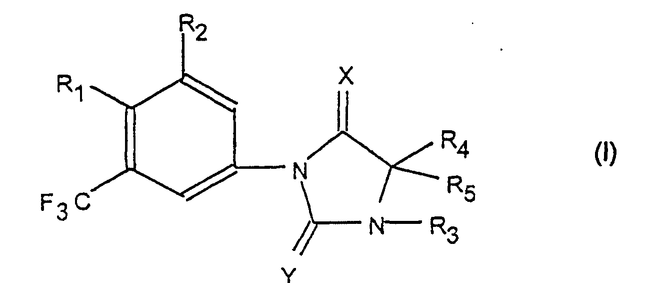 worin:
R₁ und R₂, die gleich oder verschieden sind, ausgewählt sind unter dem Wasserstoffatom, den Halogenatomen und den Alkyl-, Alkenyl-, Alkinyl-, Cyano-, Trifluormethyl-, Amino-, Monoalkylamino- und Dialkylaminoresten,
R₃ darstellt ein Wasserstoffatom, einen gegebenenfalls durch ein oder mehrere Sauerstoff- oder Schwefelatome unterbrochenen Alkylrest, einen Phenyl- oder Pyridylrest, wobei diese Reste gegebenenfalls substituiert sind mit einem oder mehreren Resten, die ausgewählt sind unter den Halogenatomen, dem Rest Phenyl, Hydroxyl, gegebenenfalls verestert, verethert oder geschützt, Alkoxy, Cyano, Trifluormethyl, Hydroxyalkyl, Carboxy in freier Form, in Ester-, Amid- oder Salzform, Amino, Mono- oder Dialkylamino, wobei das Stickstoffatom des Pyridylrests gegebenenfalls oxidiert ist,
R₄ und R₅
entweder gleich oder verschieden sind und darstellen einen Alkylrest, gegebenenfalls substituiert mit einem oder mehreren Resten, die ausgewählt sind unter den Resten Hydroxyl, gegebenenfalls verestert, verethert oder geschützt, den Halogenatomen und den Alkylthio- und Phenylthioresten, die selbst gegebenenfalls substituiert sind mit einem oder mehreren Resten, die ausgewählt sind unter den Halogenatomen und dem Hydroxylrest,
oder zusammen den Rest: 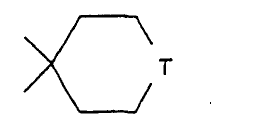 bilden, worin T ein Sauerstoff- oder Schwefelatom darstellt,
X und Y, die gleich oder verschieden sind, ein Sauerstoff- oder Schwefelatom darstellen,
wobei besagte Produkte der Formel (I) in allen möglichen racemischen, enantiomeren oder diastereoisomeren Isomerformen vorliegen, sowie die Additionssalze mit den mineralischen und organischen Säuren oder mit den mineralischen und organischen Basen besagter Produkte der Formel (I), **dadurch gekennzeichnet, dass**:
a) man ein Produkt der Formel (A): 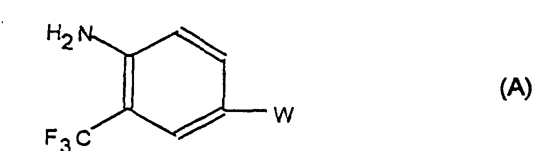 herstellt, worin W ein Halogenatom oder ein Hydantoinderivat der Formel: 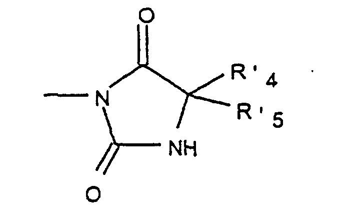 darstellt, worin R'₄ und R'₅ die oben für R₄ und R₅ angegebenen Bedeutungen haben, worin die etwaigen reaktiven Funktionen gegebenenfalls geschützt sind, indem man mit der Verbindung der Formel (II): 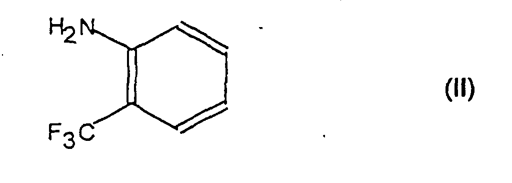 umsetzt
entweder zuerst eine Verbindung der Formel (III): 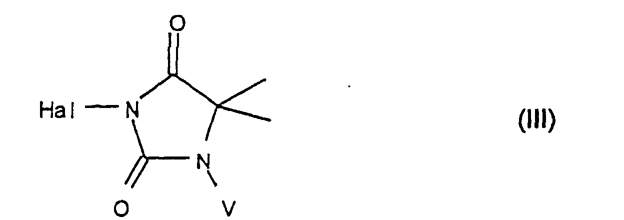 worin Hal ein Halogenatom darstellt und V ein Wasserstoffatom oder ein Halogenatom darstellt, dann die Verbindung der Formel (B): 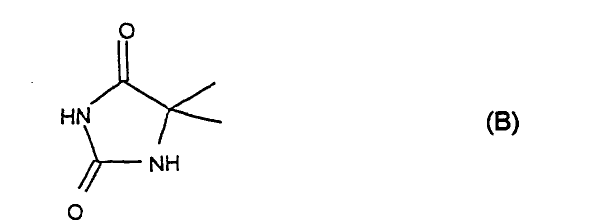 um das Produkt der Formel (A₁): 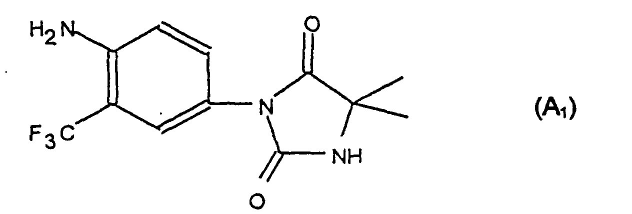 zu erhalten, das dem Produkt der Formel (A) entspricht, worin W den Dimethylhydantoinrest: 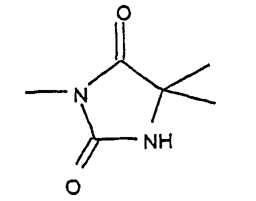 darstellt,
oder N-Bromsuccinimid in Dimethylformamid oder aber die Verbindung der Formel (III), wie oben definiert, um das Produkt der Formel (A₂): 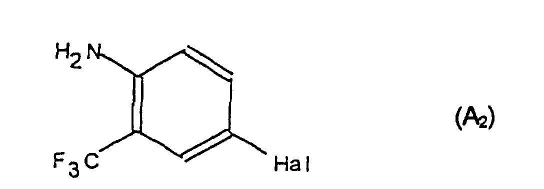 zu erhalten, worin Hal ein Bromatom oder ein anderes Halogenatom darstellt, das man mit einer Verbindung der Formel (IV): 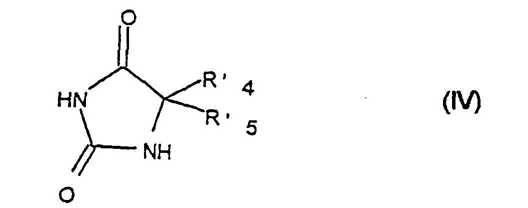 umsetzt, worin R'₄ und R'₅ die oben angegebenen Bedeutungen haben, um das Produkt der Formel (A₃): 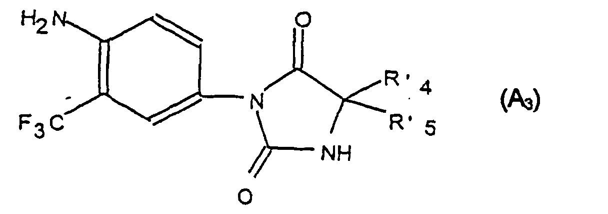 zu erhalten, das dem Produkt der Formel (A) entspricht, worin W den Rest: 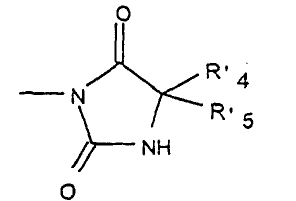 darstellt, worin R'₄ und R'₅ die oben angegebenen Bedeutungen haben,
b) man, wenn notwendig und wenn gewünscht, das so erhaltene Produkt der Formel (A) einer oder mehreren der folgenden Reaktionen in einer beliebigen Reihenfolge unterzieht:
i) einer Diazotierungsreaktion, um das Produkt der Formel (V): 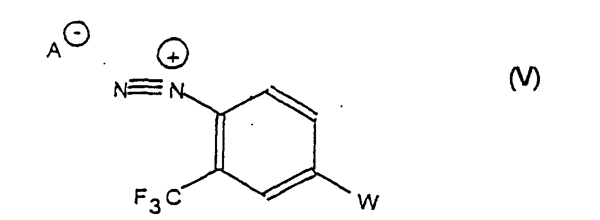 zu erhalten, worin A^{⊖} ein Anion eines Halogenatoms oder eines halogenierten Derivats darstellt und W die oben angegebene Bedeutung hat, das man einer Halogenierungsreaktion unterzieht, um das Produkt der Formel (F₁): 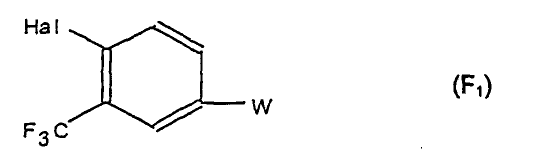 zu erhalten, worin Hal und W die oben angegebenen Bedeutungen haben, das man einer Reaktion zur Substitution am Halogenatom mit einem Metallderivat der Formel (VI):
R'₁-M (VI)
unterziehen kann, worin M ein Metall darstellt und R'₁ die oben für R₁ angegebene Bedeutung hat, worin die etwaigen reaktiven Funktionen gegebenenfalls geschützt sind,
um das Produkt der Formel (F₂): 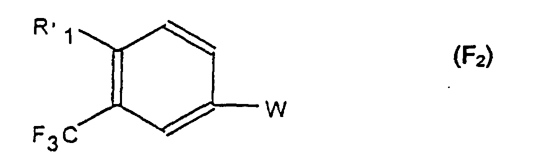 zu erhalten, worin R'₁ und W die oben angegebenen Bedeutungen haben,
ii) einer Halogenierungsreaktion, um das Produkt der Formel (F₃): 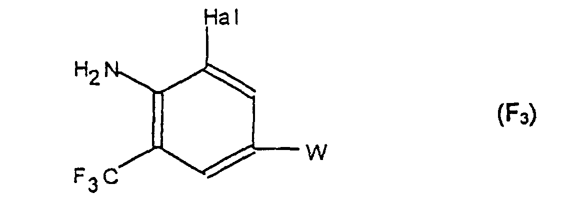 zu erhalten, worin Hal ein Halogenatom darstellt und W die oben angegebene Bedeutung hat, das man
entweder einer Reaktion zur Substitution am Halogenatom mit einem Metallderivat der Formel (VII):
R'₂-M (VII)
unterziehen kann, worin M ein Metall darstellt und R'₂ die oben für R₂ angegebene Bedeutung hat, worin die etwaigen reaktiven Funktionen gegebenenfalls geschützt sind, um das Produkt der Formel (F₄): 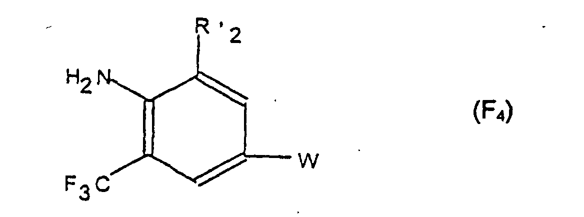 zu erhalten, worin R'₂ und W die oben angegebenen Bedeutungen haben,
das man den aufeinanderfolgenden, oben unter i) definierten Reaktionen zur Diazotierung des Aminorests, dann Halogenierung und schließlich Substitution mit einer Verbindung der Formel (VI) unterziehen kann, um das Produkt der Formel (F₅): 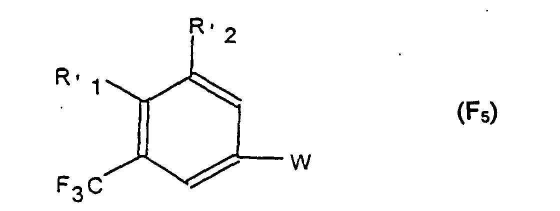 zu erhalten, worin R'₁, R'₂ und W die oben angegebenen Bedeutungen haben,
oder einer Diazotierungs-Halogenierungsreaktion unterziehen kann, um das Produkt der Formel (F₆): 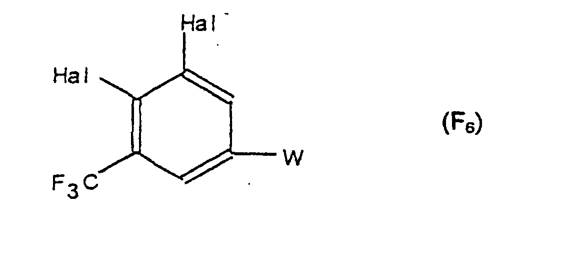 zu erhalten, worin die beiden durch Hal dargestellten Halogenatome gleich oder verschieden sind und W die oben angegebene Bedeutung hat, das man einer Reaktion zur Substitution an den Halogenatomen mit einer Verbindung der Formel (VI) oder (VII), wie oben definiert, unterziehen kann, um das Produkt der Formel (F₅), wie oben definiert, zu erhalten, worin R'₁ und R'₂ gleich sind,
c) man, wenn notwendig und wenn gewünscht, die Produkte der Formeln (F₁), (F₂), (F₃), (F₄), (F₅) und (F₆), wenn W ein Halogenatom darstellt, mit dem Produkt der Formel (IV), wie oben definiert, umsetzt, um das Produkt der Formel (I'): 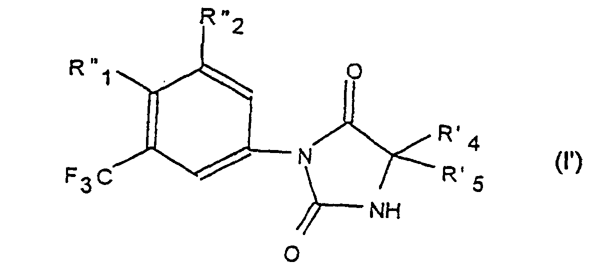 zu erhalten, worin R"₁ und R"₂ so sind, dass:
entweder R"₂ ein Wasserstoffatom darstellt
und R"₁ ein Halogenatom oder R'₁, wie oben definiert, darstellt,
oder R"₂ ein Halogenatom darstellt
und R"₁ einen Aminorest oder ein Halogenatom darstellt,
oder R"₂ R'₂, wie oben definiert, darstellt
und R"₁ einen Aminorest oder R'₁, wie oben definiert, darstellt,
d) man gegebenenfalls und wenn notwendig oder wenn gewünscht die Produkte der Formeln (A₁), (A₃) und (I') einer beliebigen oder mehreren der folgenden Reaktionen in einer beliebigen Reihenfolge unterzieht:
a) Reaktion zur Eliminierung der etwaigen Schutzgruppen, die R"₁, R"₂, R'₄ und R'₅ tragen können,
b) Reaktion zur Umwandlung einer >C=O-Gruppe in eine >C=S-Gruppe,
c) Einwirkung eines Reagenzes der Formel Hal-R'₃, worin R'₃ die Werte für R₃, wie oben definiert, hat mit Ausnahme des Wertes Wasserstoff und worin die etwaigen reaktiven Funktionen gegebenenfalls geschützt sind und Hal ein Halogenatom darstellt, um Produkte der Formel (I), wie in Anspruch 1 definiert, zu erhalten, dann, wenn gewünscht, Einwirkung eines Mittels zum Entfemen der etwaigen Schutzgruppen, die R'₃ tragen kann, oder gegebenenfalls Einwirken eines Mittels zur Veresterung, Amidbildung oder Salzbildung auf diese Produkte,
d) Reaktion zur Umwandlung eines Aminorests in einen Nitrorest.

2. Verfahren zur Herstellung, wie in Anspruch 1 definiert, der Produkte der Formel (I), wie in Anspruch 1 definiert, worin R₁ und R₂, die gleich oder verschieden sind, so sind, dass das eine ein Wasserstoffatom oder einen Cyanorest darstellt und das andere ausgewählt ist unter den Halogenatomen und den Resten Cyano und Amino,
R₃ darstellt ein Wasserstoffatom oder einen Alkylrest, gegebenenfalls substituiert mit einem Hydroxylrest, gegebenenfalls verestert, verethert oder geschützt,
R₄ und R₅, die gleich oder verschieden sind, einen linearen oder verzweigten Alkylrest mit höchstens 6 Kohlenstoffatomen darstellen, gegebenenfalls substituiert mit einem oder mehreren Resten, die ausgewählt sind unter den Resten Hydroxyl, gegebenenfalls verestert, verethert oder geschützt, und den Halogenatomen, und X und Y ein Sauerstoffatom darstellen.

3. Verfahren zur Herstellung, wie in Anspruch 1 definiert, der folgenden Produkte:
- 3-[4-Amino-3-(trifluormethyl)phenyl]-5,5-dimethyl-2,4-imidazolidindion,
- 5,5-Dimethyl-3-(4-iod-3-(trifluormethyl)phenyl)-2,4-imidazolidindion,
- 4-(4,4-Dimethyl-2,5-dioxo-3-(4-hydroxybutyl)-1-imidazolidinyl)-2-(trifluormethyl)-benzonitril,
- 4-(2,4-Dioxo-1-(4-hydroxybutyl)-8-oxa-1,3-diazaspiro(4,5)decan-3-yl)-2-(trifluormethyl)benzonitril,
- 5,5-Dimethyl-3-(4,5-dicyano-3-(trifluormethyl)phenyl)-2,4-imidazolidindion,
wobei diese Produkte in allen möglichen racemischen, enantiomeren und diastereoisomeren Isomerformen vorliegen, sowie ihre Additionssalze mit den mineralischen und organischen Säuren oder mit den mineralischen und organischen Basen, die pharmazeutisch annehmbar sind.

4. Verfahren zur Herstellung der Produkte der Formel (I), wie in den Ansprüchen 1 bis 3 definiert, **dadurch gekennzeichnet, dass** man, um das Produkt der Formel (A₁) aus den Produkten der Formeln (II), (III) und B, wie in Anspruch 1 definiert, zu erhalten, in einem Lösungsmittel, das ausgewählt ist unter Dimethylsulfoxid, Triglyme, Dimethylacetamid oder Dimethylformamid und vorzugsweise Dimethylacetamid arbeitet.

5. Verfahren zur Herstellung der Produkte der Formel (I), wie in Anspruch 4 definiert, **dadurch gekennzeichnet, dass** die Verbindung der Formel (III) das Dibromderivat der Formel: 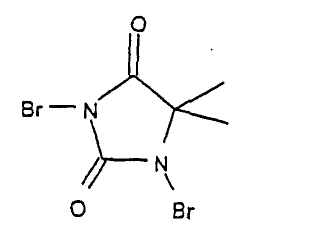 ist und dass man ein halbes Mol dieser Verbindung und ein halbes Mol der Verbindung der Formel B auf ein Mol der Verbindung der Formel (II) verwendet.

6. Verfahren zur Herstellung der Produkte der Formel (I), wie in den Ansprüchen 4 und 5 definiert, **dadurch gekennzeichnet, dass** die Reaktion bei einer Temperatur von 130°C bis 160°C und vorzugsweise bei 155°C durchgeführt wird.
